# EUROPEAN PATENT SPECIFICATION

(11) **EP 3 789 019 B1**
(45) Date of publication and mention of the grant of the patent: **23.08.2023**
(21) Application number: 18917117.6
(22) Date of filing: 09.11.2018
(51) Int. Cl.: A61K 31/137, A61K 31/155, A61K 31/4418, A61K 36/61, A61K 36/82, A61K 36/899, A61K 8/41, A61K 8/43, A61K 8/49, A61K 8/92, A61K 8/9794, A61K 9/00, A61K 9/08, A61K 9/10, A61P 17/00, A61P 17/02, A61P 31/02, A61P 31/10, A61Q 19/00, A61K 36/89

(54) **COMPOSITIONS COMPRISING CICLOPIROX OLAMINE FOR THE PREVENTION OR TREATMENT OF SKIN INFECTIONS**
ZUSAMMENSETZUNG ENTHALTEND CICLOPIROX OLAMIN ZUR VORBEUGUNG ODER BEHANDLUNG VON HAUTINFEKTIONEN
COMPOSITIONS CONTENANT DU CICLOPIROX OLAMINE DESTINÉES À LA PRÉVENTION OU AU TRAITEMENT DES INFECTIONS CUTANÉES

(30) Priority: 03.05.2018 KR 20180051150
(43) Date of publication of application: 10.03.2021
(73) Proprietor: Careside Co., Ltd., Gyeonggi-do 13209 (KR)
(72) Inventor: YOU, Young Kook, Seoul 06990 (KR)
(74) Representative: Taruttis, Tilman
(86) International application number: PCT/KR2018/013614
(87) International publication number: WO 2019/212110

(56) References cited:
- KR-B1- 101 658 361
- KR-B1- 101 658 365
- KR-B1- 101 899 413
- US-A1- 2017 290 778
- US-B1- 6 437 002
- HEALY BRENDAN ET AL: "Topical and oral treatments for fungal skin infections", PRESCRIBER, vol. 17, no. 7, 5 April 2006 (2006-04-05), pages 30-43, XP055863939, ISSN: 0959-6682, DOI: 10.1002/psb.360
- SHINO BEENA ET AL: "Comparison of Antimicrobial Activity of Chlorhexidine, Coconut Oil, Probiotics, and Ketoconazole on Candida albicans Isolated in Children with Early Childhood Caries: An In Vitro Study", SCIENTIFICA, vol. 2016, 14 March 2016 (2016-03-14), pages 1-5, XP055863943, US ISSN: 2090-908X, DOI: 10.1155/2016/7061587
- ADITYA K GUPTA ET AL: "Ciclopirox for the treatment of superficial fungal infections: a review", INTERNATIONAL JOURNAL OF DERMATOLOGY, WILEY-BLACKWELL PUBLISHING LTD, UK, vol. 42, 25 June 2010 (2010-06-25), pages 3-9, XP071186520, ISSN: 0011-9059, DOI: 10.1046/J.1365-4362.42.S1.2.X

## Description

### BACKGROUND

### 1. Field

The present invention relates to a composition for preventing or treating skin infections.

### 2. Description of the Related Art

Since the skin covers the surface of the body, there are many opportunities for direct contact with various pathogens, such as dermatophytes and Candida, and being infected. In addition, since various bacteria, fungi and yeasts are parasitic on the skin and fur of the animal, skin diseases frequently appear.

Pathogenic bacteria that cause various skin diseases in the skin of animals include Propionibacterium acnes, Borelia burgdorferi, staphilococcus aureus, Pseudomonas aeruginosa and Proteus mirabilis, and pathogenic fungi include Microsporium canis, Microsporium gypseum and Trichophyton mentagrophytes.

Currently, Ampotericin B, clotrimazole, crinipan AD, fluconazole, griseofulvin or ketoconazole is used as an antifungal agent for treating skin infections caused by these pathogenic fungi, and triclinicacid, triclocarban, benzalkonium chloride or benzetonium chloride is used as an antibacterial agent.

However, these antifungal agents or antimicrobial agents are difficult to purify, costly to manufacture, and have problems such as increased resistance to microorganisms, and do not exhibit antimicrobial effects in small amounts. Further, skin infections appear from a complex factor such as bacteria, fungi and yeast, and thus have a disadvantage in that they do not exhibit antimicrobial activity against all of them.

KR-B-101658 361 discloses combinations comprising terbinafine hydrochloride, chlorhexidine gluconate, malic acid and sodium borate having a pH value in the range of 5.3 to 5.7 for use in the prevention and treatment of skin infections.

### SUMMARY

The invention discloses a composition for preventing or treating skin infections, which is excellent in the treatment effect of skin infections due to a wide antibacterial spectrum, has less side effects, and does not generate a precipitate.

In a first embodiment, pharmaceutical compositions according to claim 1 are provided.

In another embodiment, the compositions of the present invention further include one or more of tea tree oil, oat extract, phytosphingosine, salts of phytosphingosine, ceramides and ceramide derivatives.

In yet a further embodiment, cosmetic compositions according to claim 9 are provided.

Other specific details of the invention are included in the detailed description and drawings.

The composition for preventing or treating skin infections according to an embodiment of the present disclosure does not generate a precipitate.

The composition for preventing or treating skin infections according to an embodiment of the present disclosure is excellent in antimicrobial activity but less toxic to the skin and does not cause side effects even after long-term use.

The composition for preventing or treating skin infections according to an embodiment of the present disclosure has a broad antimicrobial spectrum up to pathogenic bacteria, pathogenic fungi and yeasts.

### BRIEF DESCRIPTION OF THE DRAWINGS

These and/or other aspects will become apparent and more readily appreciated from the following description of the embodiments, taken in conjunction with the accompanying drawings in which:
FIGS. 1 to 3 are results of the formation of the inhibitory band, which are the antimicrobial activity test results of the composition according to the present specification for Candida albicans.
FIGS. 4 and 5 are graphs showing the antimicrobial activity test results of the composition according to the present specification for Candida albicans.
FIGS. 6 to 15 are results of the formation of inhibitory band, which are the antimicrobial activity test results of the composition according to the present specification for Candida parapsilosis.
FIGS. 16 and 17 are graphs showing the antimicrobial activity test results of the composition according to the present specification for Candida parapsilosis.
FIGS. 18 to 27 are results of the formation of the inhibitory band, which are the antimicrobial activity test results of the composition according to the present specification for Candida krusei.
FIGS. 28 and 29 are graphs showing the antimicrobial activity test results of the composition according to the present specification for Candida krusei.
FIGS. 30 to 39 are results of the formation of the inhibitory band, which are the antimicrobial activity test results of the composition according to the present specification for Aspergillus fumigatus.
FIGS. 40 and 41 are graphs showing the antimicrobial activity test results of the composition according to the present specification for Aspergillus fumigatus.

### DETAILED DESCRIPTION

Advantages and features of the inventions disclosed herein, and methods of achieving them will become apparent with reference to the embodiments described below in detail in conjunction with the accompanying drawings. However, the present disclosure is not limited to the embodiments disclosed below, but may be implemented in various different forms, and the embodiments are only provided to make the disclosure of the present disclosure complete. The scope of the present invention is defined by the claims

The terminology used herein is for the purpose of describing particular embodiments only and is not intended to be limiting of the scope of the present disclosure. In this specification, the singular also includes the plural unless specifically stated otherwise in the phrase. As used herein, "comprises" and/or "comprising" does not exclude the presence or addition of one or more other components in addition to the mentioned components. Like reference numerals refer to like components throughout the specification, and "and/or" includes each and all combinations of one or more of the mentioned components.

Unless otherwise defined, all terms used in the present specification (including technical and scientific terms) may be used in a sense that can be commonly understood by those skilled in the art to which this specification belongs. Further, terms that are defined in a commonly used dictionary are not ideally or excessively interpreted unless they are specifically defined clearly.

Hereinafter, the present invention will be described in detail so that those skilled in the art can clearly understand.

The composition for preventing or treating skin infections according to the present specification is defined in claims 1 and 9. Skin infections according to the present specification mainly mean skin infections caused by pathogens, and include skin diseases caused by various bacteria, fungi and yeasts.

Terbinafine hydrochloride [(2E)-6,6-dimethylhept-2-en-4-yn-1-yl] (methyl) (naphthalen-1-ylmethyl)aminehydrochloride) is represented by the following Chemical Formula 1, and is a white crystalline powder, which can be dissolved in methanol, dichloromethane, ethanol and water:

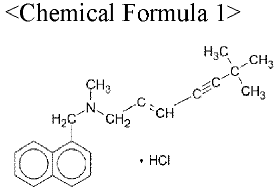

The terbinafine hydrochloride inhibits the synthesis of ergosterol by inhibiting squalene epoxidase, which is one of the enzymes involved in the synthesis of fungal cell membranes. This causes a change in the permeability of the cell membrane and lyses the fungus to act as an antifungal action.

The terbinafine hydrochloride is included in an amount 0.1 to 2 parts by weight, based on the composition. When included with the above range, the antibacterial activity is excellent, but there is little irritation to the skin and toxicity does not appear even after long-term use. Further, in the case of including terbinafine hydrochloride in the above range, it can further prevent the generation of a precipitate by further including malic acid and sodium borate described later.

Chlorhexidine gluconate is represented by the following Chemical Formula 2.

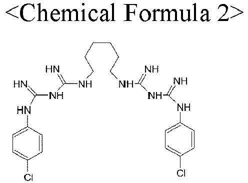

The chlorhexidine gluconate is present in an amount of 0.1 to 4.0 parts by weight and exhibits antimicrobial activity against various bacteria and fungi, including gram-positive and gram-negative bacteria. The chlorhexidine gluconate is a liquid substance, in which gluconic acid and chlorhexidine are combined. The chlorhexidine tends to react with other anions than gluconic acid to form precipitates.

Since most liquid medicines include excipients that include chloride ions, the chloride ions can be combined with the chlorhexidine to generate a precipitate. Moreover, when the chlorhexidine gluconate is used in a complex formulation with terbinafine hydrochloride, a precipitate may be generated by the combination of chlorhexidine and chloride ions of terbinafine hydrochloride.

The composition for preventing or treating skin infections further comprises malic acid and sodium borate. In an exemplary embodiment, the composition for preventing and treating skin infection has a pH value in the range of 5.3 to 5.7.

Malic acid is one of the polycarboxylic acids and can serve as an ion scavenger and prevent the generation of precipitates. As can be seen through the experimental example described below, malic acid may play a role of preventing a precipitate derived from chlorohexidine gluconate, unlike citric acid, oxalic acid, and tartaric acid. Malic acid is included in an amount of 0.5 to 2.0 parts by weight based on the composition.

Sodium borate can prevent the formation of a precipitate, unlike sodium chloride, ammonia water, sodium citrate, ammonium carbonate, as can be confirmed through the experimental example described later. Sodium borate can adjust the pH of a composition that is altered by maleic acid.

Sodium borate is included in an amount of 0.3 to 0.5 parts by weight based on the composition. The chlorhexidine gluconate is included in an amount of 0.1 to 4 parts by weight, based on the composition. When included with the above range, it shows excellent antibacterial activity and makes less irritation to the skin. Further, when including the chlorohexidine gluconate in the above range, it may prevent the generation of a precipitate by further including malic acid and sodium borate.

The ciclopirox olamine (C12H17NO2•C2H7NO, [2(1H)-Pyridinone, 6-cyclohexyl-1-hydroxy-4-methyl-compound with 2-aminoethanol (1: 1)]) is represented by the following Chemical Formula 3, and is an olamine salt form of ciclopirox

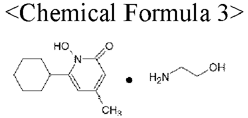

Ciclopirox olamine is a synthetic antimicrobial agent with antibacterial and anti-inflammatory action with a synthetic antimicrobial action. Ciclopirox exerts its action by inhibiting the availability of cofactors essential for enzymes by binding and chelating trivalent cations such as Fe³⁺ and Al³⁺. This can result in the loss of activity of enzymes essential for cell metabolism, organization of cell wall structures and other important cellular functions. Ciclopirox can also exhibit anti-inflammatory activity by inhibiting 5-lipoxygenase and cyclooxygenase (COX).

The ciclopirox olamine is included in an amount of 0.1 to 6 parts by weight, or 1 to 5 parts by weight, based on the composition.

Further, when used in combination with the terbinafine hydrochloride and chlorhexidine gluconate, it shows activity against pathogenic bacteria, fungi and yeast while not showing activity when used alone, and shows excellent antibacterial activity even when used in small amounts, and thus irritation to the skin can be reduced.

Since various microorganisms, such as pathogenic bacteria, fungi and yeast, are parasitic on the skin of animals, killing only bacteria can activate fungi, or killing fungi can activate bacteria, which causes secondary diseases. When the combination of terbinafine hydrochloride, chlorhexidine gluconate, and ciclopirox olamine is used, the spectrum of antimicrobial activity to bacteria, fungi, and yeast is broadened, and thus, even when a small amount is used, it shows excellent antimicrobial activity, thereby reducing skin irritation and being more suitable for animals. In addition, when used as an external preparation for skin, it can also exert whitening action, and thus the composition according to the present specification may be applied to cosmetic compositions as well as pharmaceutical compositions for preventing or treating skin infections.

The composition for preventing or treating skin infections of the present invention may further comprise one or more of tea tree oil, oat extract, phytosphingosine, salts thereof, ceramide and ceramide derivatives.

The tea tree oil has antibacterial properties, especially when combined with terbinafine hydrochloride, chlorhexidine gluconate and ciclopirox olamine, the antimicrobial activity is further increased, and it is a natural extract, which does not irritate the skin and does not show any side effect even after long-term use. It also stabilizes the edema caused by the infection and helps relieve erythema.

The tea tree oil refers to a pale yellow essential oil obtained from a tea tree named Melaleucaalternifolia.

The tea tree oil may be prepared according to a conventional method. Specifically, the tea tree oil may be obtained by separating a solution obtained by extracting the tip of a fresh leaf or a branch of the tea tree by steam distillation.

The tea tree oil contains more than 48 kinds of ingredients, mainly terpene ingredients, such as 1-terpinen-4-ol, α-pinene, α-terpinene. ρ-cimen, γ-terpinene, terpinolene, α-terpineol and 1,8-cineol.

The tea tree oil may be included in an amount of 0.1 to 10 parts by weight, and preferably 1 to 5 parts by weight, based on the composition. When included with the above range, the antimicrobial effect of the composition of the present invention can be increased and the symptoms of skin infections such as erythema can be alleviated while inhibiting the overgrowth of microorganisms other than pathogenic microorganisms.

The oat extract may relieve skin itching due to infection and may impart moisture to the skin. Further, since it is a natural extract, there are less side effects even after long-term use, and when used in combination with terbinafine hydrochloride and chlorhexidine gluconate, the treatment and prevention effect of skin infections caused by pathogens can be further enhanced.

The oat extract is obtained from oat (Avena sativa), which is cereals. Oat is rich in minerals, such as high quality protein, phosphorus, magnesium and calcium. The oat extract can be obtained by extracting with the conventional grain extract manufacturing method.

Specifically, the oat extract can be extracted from the ground product obtained by drying or grinding the oats with a solvent selected from the group including distilled water, an organic solvent and a mixture thereof, and two or more solvents may be sequentially used depending on the polarity of the organic solvent. The organic solvent known in the art may be used without limitation, specifically, lower alcohols such as methanol, ethanol, isopropyl alcohol, butanol, polyhydric alcohols such as glycerol, ethylene glycol, propylene glycol, 1, 3-butylene glycol, and alone or a mixture thereof selected from a group including hydrocarbon solvent such as petroleum ether, methyl acetate, ethyl acetate, benzene, hexane, methylene chloride, diethyl ether, dichloromethane, chloroform, acetone may be used. Preferably, it can be extracted using water, ethanol or mixtures thereof to reduce irritation and toxicity to the skin.

The content of the solvent during the extraction may be added in an amount of 1 to 15% by volume based on the dry weight of the ground product and then a warm immersion method of extracting by heating at 50 to 100 ° C for 1 to 24 hours, or a cold immersion method of depositing at a low temperature of 4 to 25 ° C for 1-20 days may be used. The extract filtrate obtained at this time is dehydrated or desolvated, and then concentrated under reduced pressure or lyophilized, and then blended into the composition for preventing or treating skin infection of the present invention in liquid or powder form.

The oat extract may be included in an amount of 0.1 to 10.0 parts by weight, and preferably 1 to 5 parts by weight based on the composition. If it is included with the above range, the effect of using the oat extract can be obtained and there is no difficulty in various formulating due to good feeling of use.

The phytosphingosine has a molecular formula of C₁₈H₃₉NO₃ and is represented by the following Chemical Formula 4:

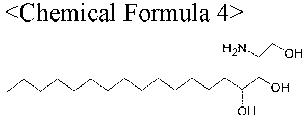

The phytosphingosine is produced by the decomposition of ceramide on the skin surface and is present in the stratum corneum by about 1 to 2%. The phytosphingosine exerts the antibacterial action against external pathogens and may further increase the antibacterial activity of the composition of the present invention when used in combination with the terbinafine hydrochloride and chlorhexidine gluconate. Further, by promoting the synthesis of ceramide may reduce the inflammation caused by skin infections and improve the skin regeneration ability to play a role in the rapid recovery of skin wounds.

The phytosphingosine may be included in an amount of 0.005 to 1 parts by weight, and preferably 0.005 to 0.1 parts by weight based on the composition. When included with the above range, it can improve the skin regeneration ability and increase the antibacterial ability to achieve the purpose of using phytosphingosine, and there is no difficulty in formulating.

The phytosphingosine may be used in the form of its salt and the like, such as phytosphingosine hydrochloride.

The ceramide is the most important lipid of the intercellular lipids (total 40-50%) to prevent moisture evaporation and to maintain moisture to serve to provide moisture to the skin. Further, by improving the function of the skin protective barrier of the stratum corneum, it prevents skin infections and heals skin infections faster.

The ceramide derivatives increase the regenerative capacity of the skin to further increase the skin infection treatment effect as well as provide moisture to the skin. The ceramide derivatives may use alone or a mixture thereof selected from a group including Bishydroxyethyl biscetyl maloamide, Hydroxypropyl bispamitamide MEA, Hydroxypropyl bisuraramide MEA, Hydroxypropyl bisstearoylamide MEA, and Hydroxypropyl bisstearoylamide MEA according to lipophilic chain length and hydrophilic.

The ceramide or ceramide derivative may be included in an amount of 0.005 to 1 parts by weight, and preferably 0.005 to 0.1 parts by weight based on the composition. When included with the above range, it can improve the skin regeneration ability while improving the therapeutic effect for the skin infection to achieve the object of the present invention including ceramide or ceramide derivatives, and there is no difficulty in formulating.

The composition for preventing or treating a skin infection of the present invention may be formulated as an external preparation for skin as a pharmaceutical composition. At this time, in addition to the above-mentioned effective ingredient, the one or more pharmaceutically acceptable carrier may be further included for administration. As the pharmaceutically acceptable carrier, alone or a mixture thereof selected from a group including saline, sterile water, Ringer's solution, buffered saline, dextrose solution, maltodextrin solution, glycerol and ethanol may be used, if necessary, other conventional additives, such as an antioxidant, buffer and bacteriostatic agent, can be added. It may also be formulated by additionally adding diluents, dispersants, surfactants, binders and lubricants. Furthermore, it may be preferably formulated according to each disease or component by a suitable method in the art or using a method disclosed in Remington's Pharmaceutical Science (Recent Edition), Mack Publishing Company and Easton PA.

The pharmaceutical compositions of the present invention are preferably administered topically and may be provided in the form of ointments, creams, emulsions, plasters, powders, impregnation pads, solutions, gels, sprays, lotions or suspensions for the treatment of skin and mucous membranes.

The preferred dosage of the pharmaceutical composition of the present invention depends on the patient's condition, condition, extent of disease, form of drug, route of administration and duration, but may be appropriately selected by those skilled in the art. Preferably it is applied to the affected area several times at about 10-30 ml or 10-30 g each time.

The composition of the invention for treating and preventing skin infections may be prepared with cosmetic compositions. The cosmetic composition of this invention can mix and blend the various components typically used for cosmetics in the range which does not impair the objective of this invention other than the said component. Specifically, for example, natural animal and vegetable fats and oils such as lanolin and squalene, higher alcohols such as stearyl alcohol and isostearyl alcohol, moisturizing agents such as higher fatty acids, glycerin, hyaluronic acid, vitamin C, vitamin E, ultraviolet ray absorbers, and ultraviolet ray shielding agents, preservatives, viscosity modifiers, pigments, fragrances and the like can be blended in any amount as required by those skilled in the art.

The cosmetic composition may have a formulation such as lotion, cream, cleansing cream, cleansing foam, cleansing water, powder, essence, pack, ointment, soap or shampoo.

Hereinafter, the present invention will be described in more detail through experimental examples.

### <Experiment Overview>

1. Experimental method: modified clsi method
2. Indicator bacteria and conditions (Table 01)

**[Table 1]**

| KCCM | ATCC | Strain Name | Used Medium | Culture Temperature |
|---|---|---|---|---|
| 60450 | 28188 | Trichophyton rubrum | Sabouraud's agar | 30°C |
| 11894 | 12078 | Malassezia furfur | YM | 30°C |
| | | Candida albicans | YM | 30°C |
| 51287 | | Candida tropicalis | YM | 30°C |
| 50701 | | Candida glabrata | YM | 25°C |
| 50030 | | Candida parapsilosis | YM | 24°C |
| 11655 | | Candida krusei | YM | 24°C |
| 50544 | 2344 | Cryptococcus neoformans | YM | 26°C |
| 60044 | 16883 | Aspergillus flavus | Czapek | 24°C |
| 60072 | | Microsporum canis | Sabouraud's agar | 28°C |
| 60331 | 96918 | Asoergillus fumigatus | PDA | 24°C |
| 60449 | 28185 | Trichophyton mentagrophytes | Sabouraud's agar | 25°C |

3. Experimental samples: existing antimicrobial agents, 63 new samples (Table 02)

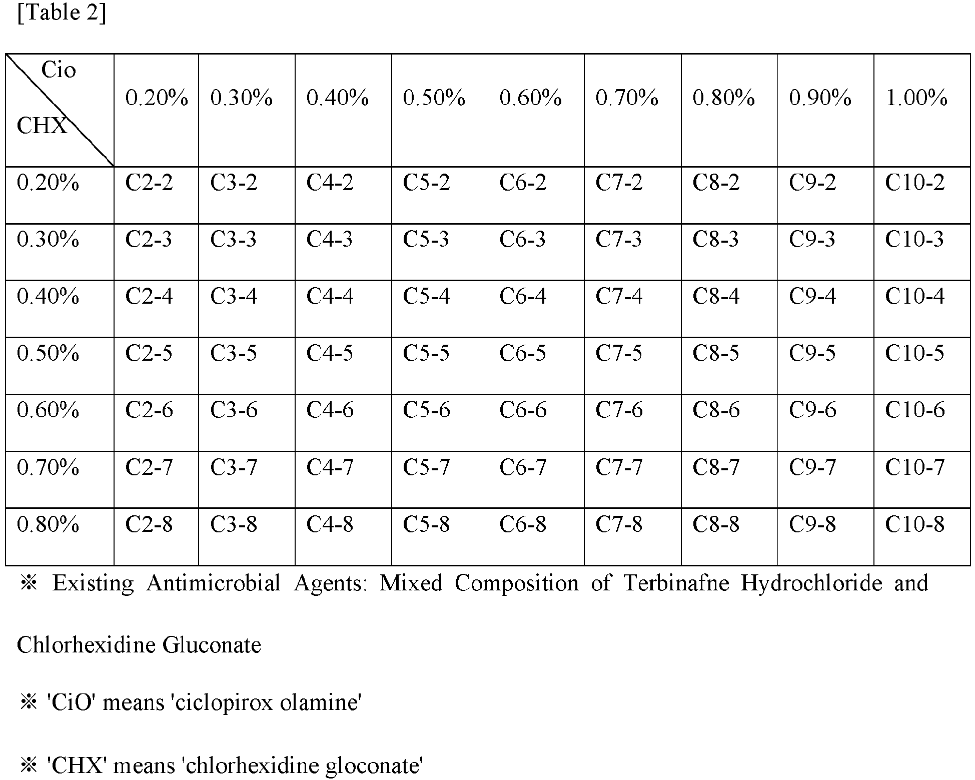

### <Experiment Result>

The experiment was conducted while using Candida albicans, Candida prapsilosis, Candida krusei and Aspergillus fumigatus as an indicator to determine the antimicrobial activity of the composition according to the present specification.

### 1. Candida albicans

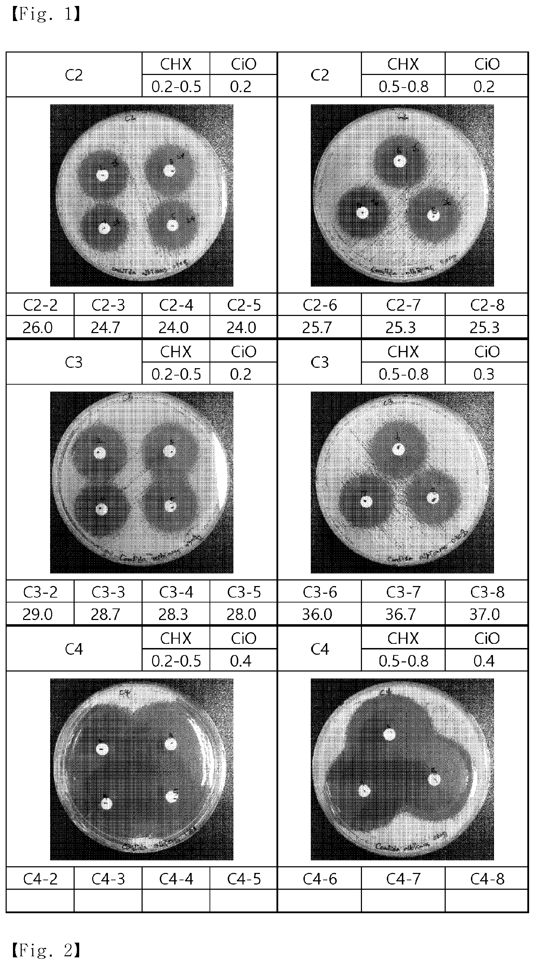

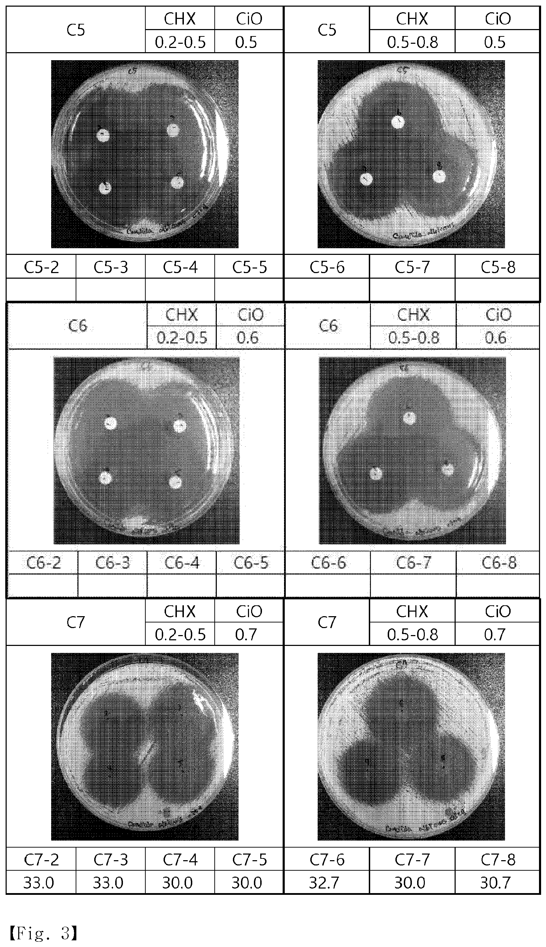

FIGS. 1 to 3 are results of the formation of the inhibitory band, which are the antimicrobial activity test results of the composition according to the present specification for Candida albicans.

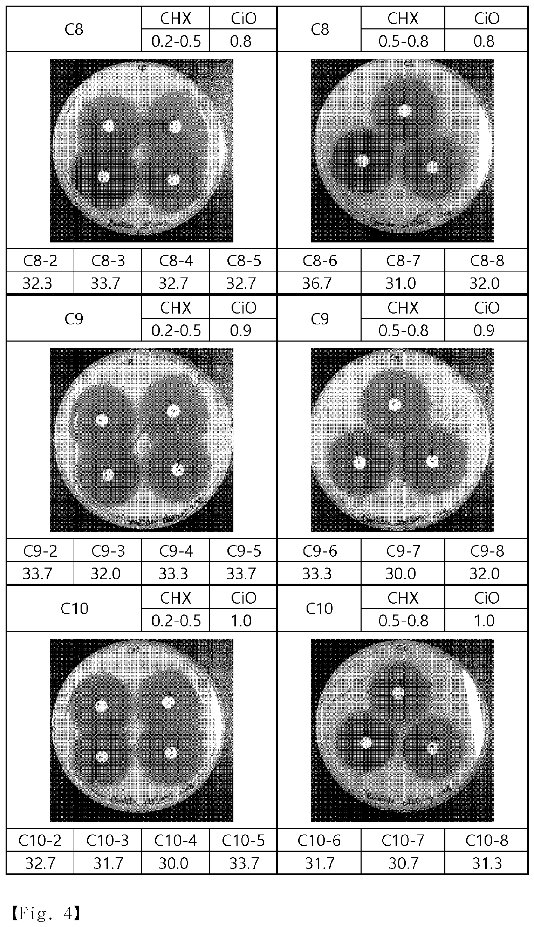

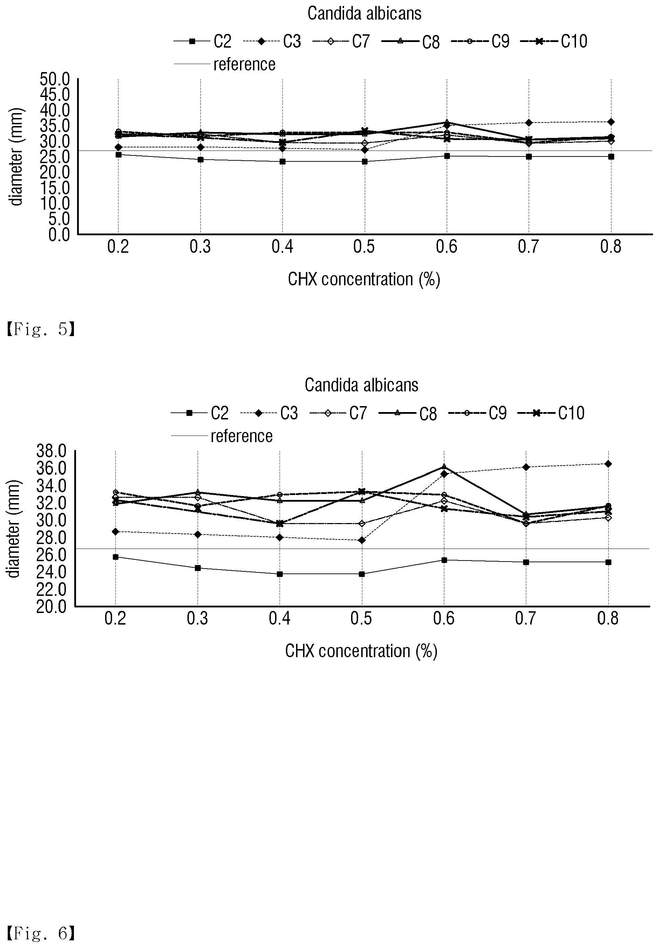

FIGS. 4 and 5 are graphs showing the antimicrobial activity test results of the composition according to the present specification for Candida albicans.

Referring to FIGS. 4 and 5, the size of the antimicrobial activity inhibitory band of the existing product is indicated by a red line to facilitate the comparative analysis. FIG. 5 is an enlarged graph of a portion of the inhibitory band (y-axis) in FIG. 4.

The Candida albicans is a strain involved in candidiasis, atopic dermatitis, etc. of an animal, and the inhibitory band of the existing product in the strain was found to be 27 mm, and the new sample at all concentrations except the sample having 0.2% CiO concentrations has larger inhibitory bands formed than the existing product. In particular, in the case of sample C3 having 0.3% CiO concentrations, a large inhibitory band of 34 mm or more was formed from a sample having CHX concentrations of 0.6% or more, and the value tends to be increased in a concentration-dependent manner. However, the rest of the samples except C3 showed a tendency to decrease the size of the inhibitory band from more than 0.6% of CHX concentrations, and overall the size of the inhibitory band increased with increasing CiO concentrations. Unusually, although not specified in the graph, there was a sudden increase in the inhibitory band in the samples from C4 to C6.

### 2. Candida parapsilosis

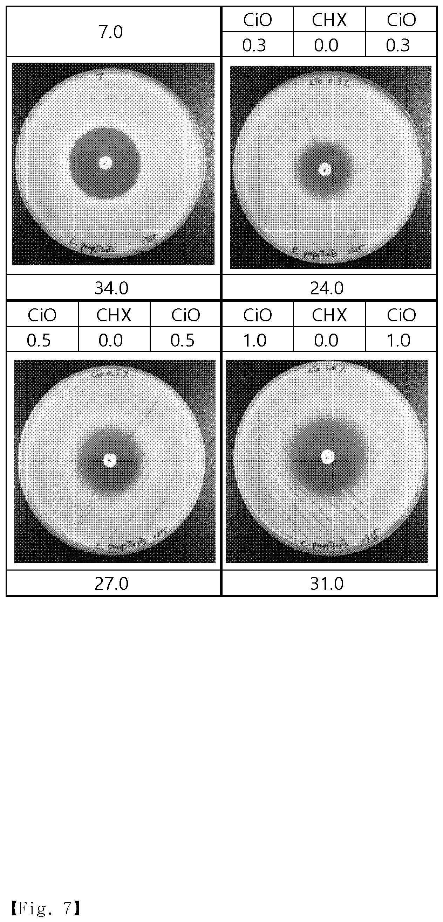

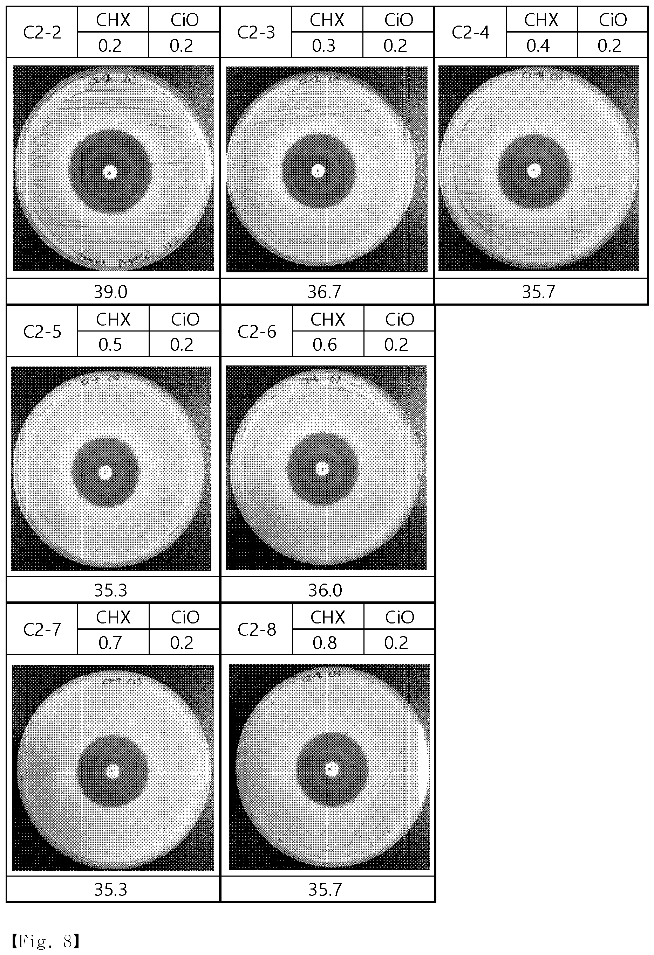

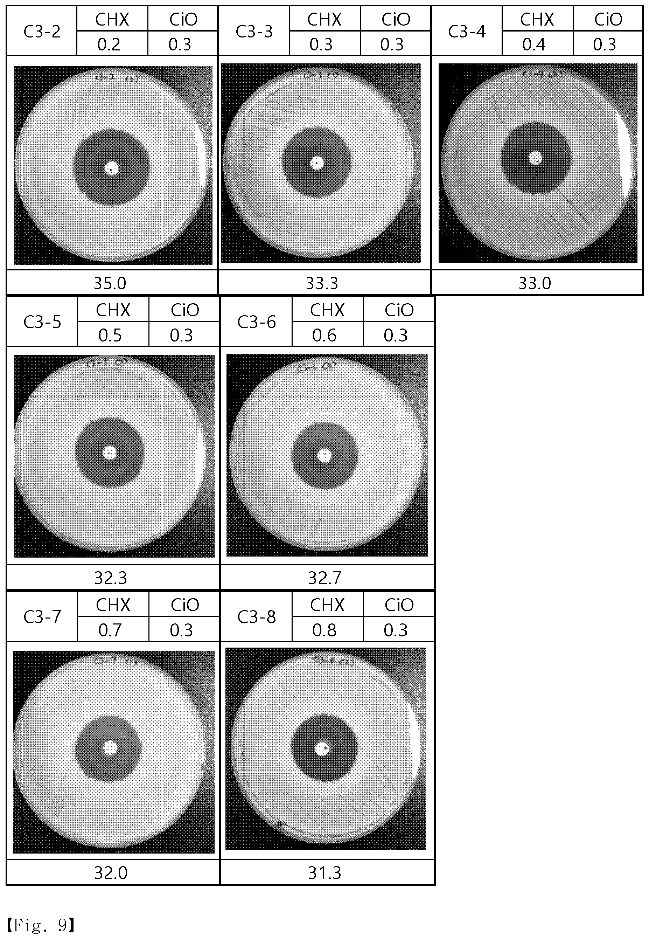

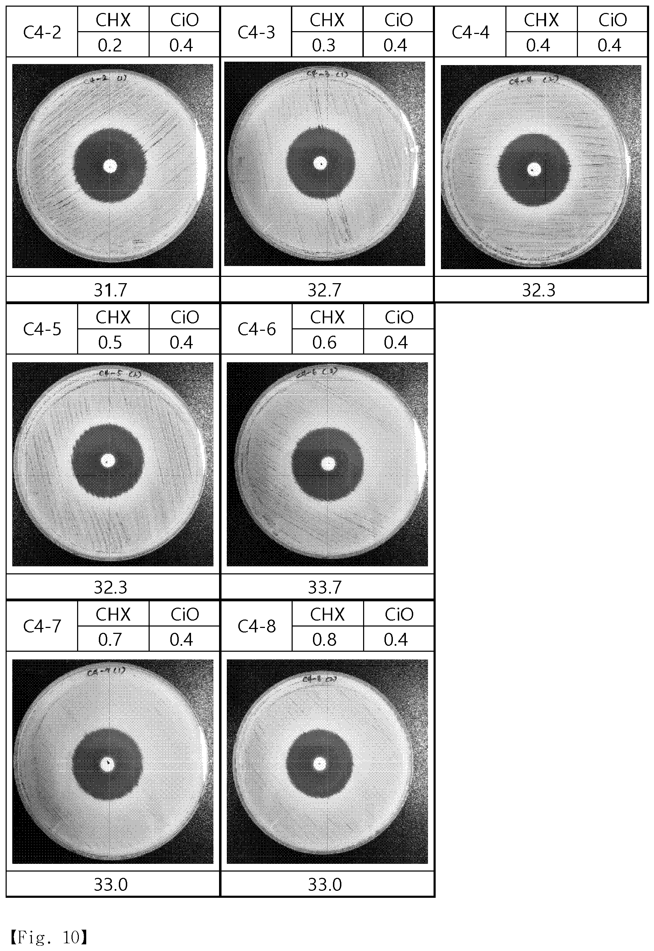

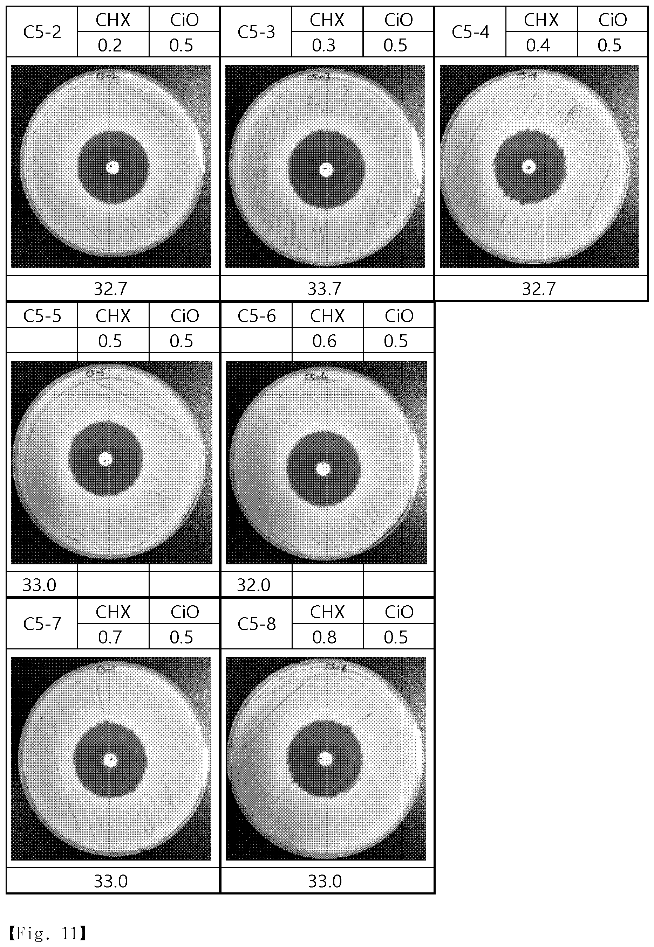

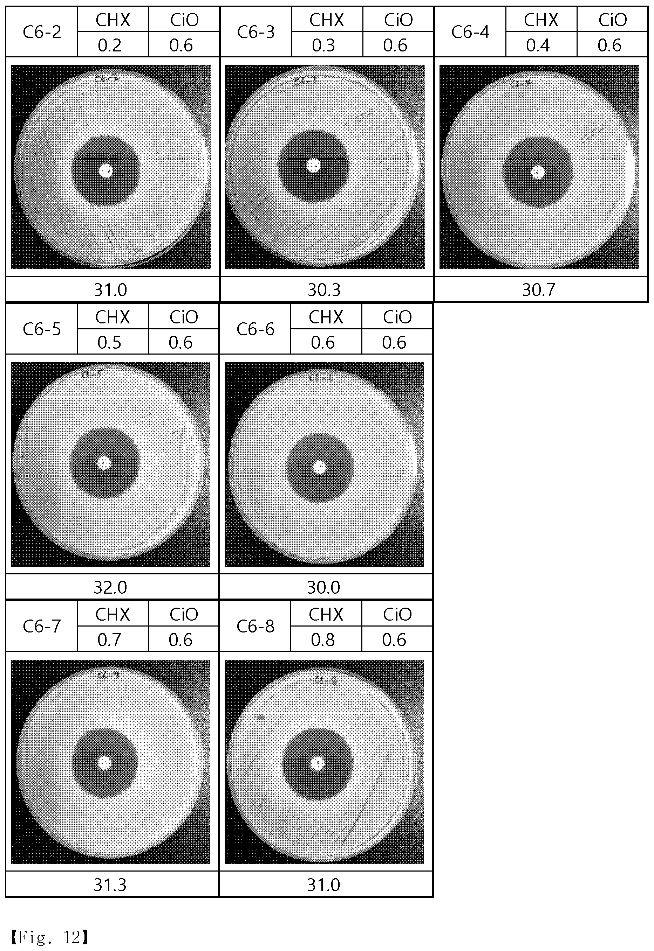

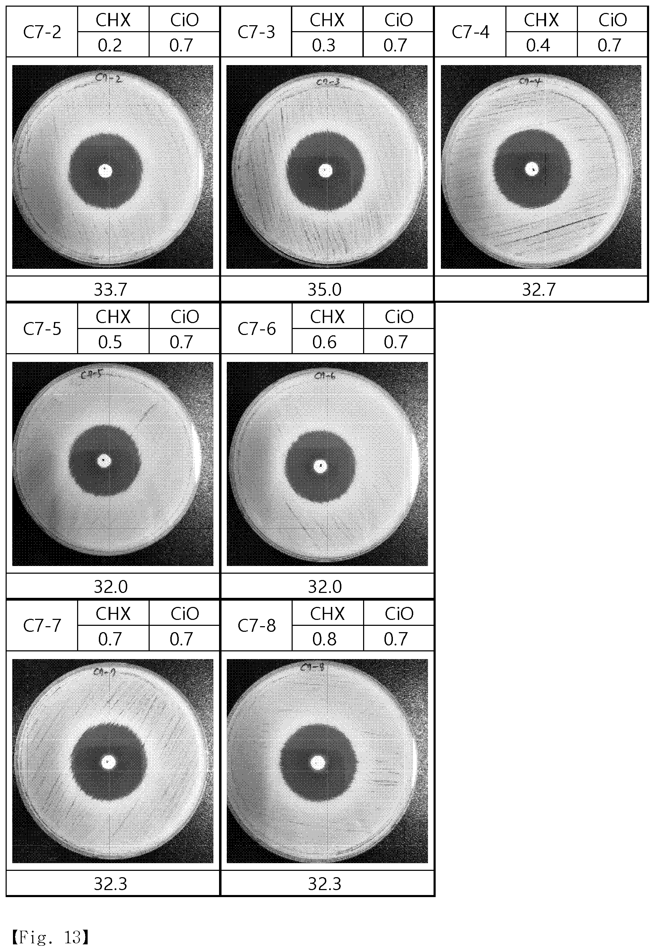

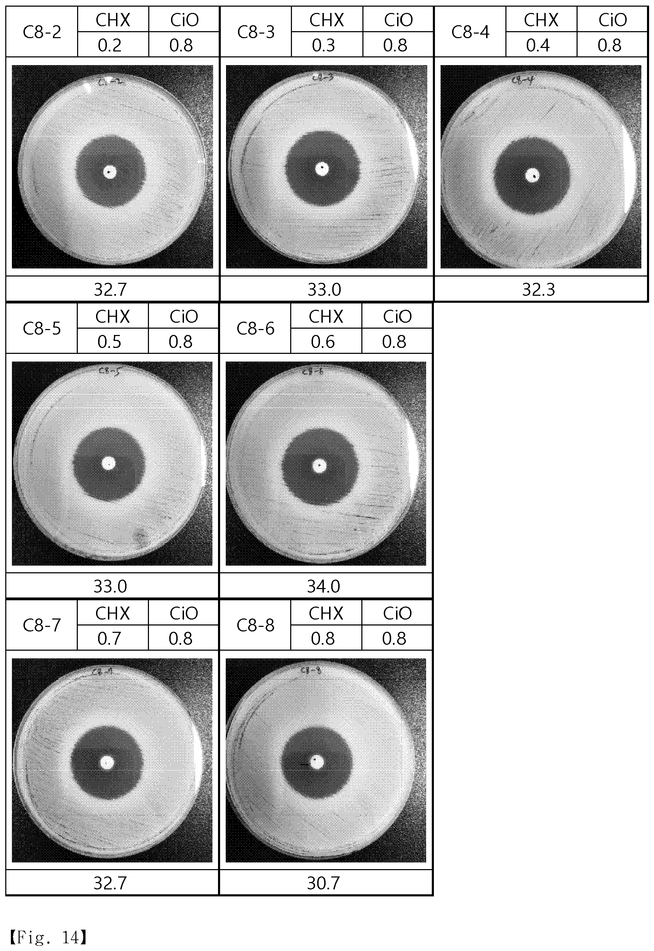

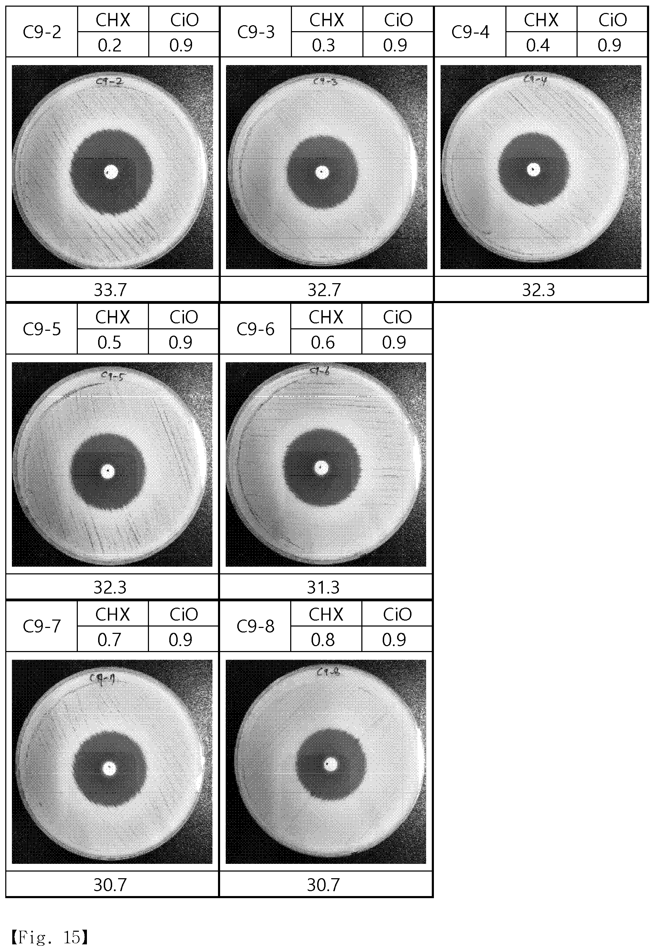

FIGS. 6 to 15 are results of the formation of inhibitory bands, which are antimicrobial activity test results of the composition according to the present specification for Candida parapsilosis.

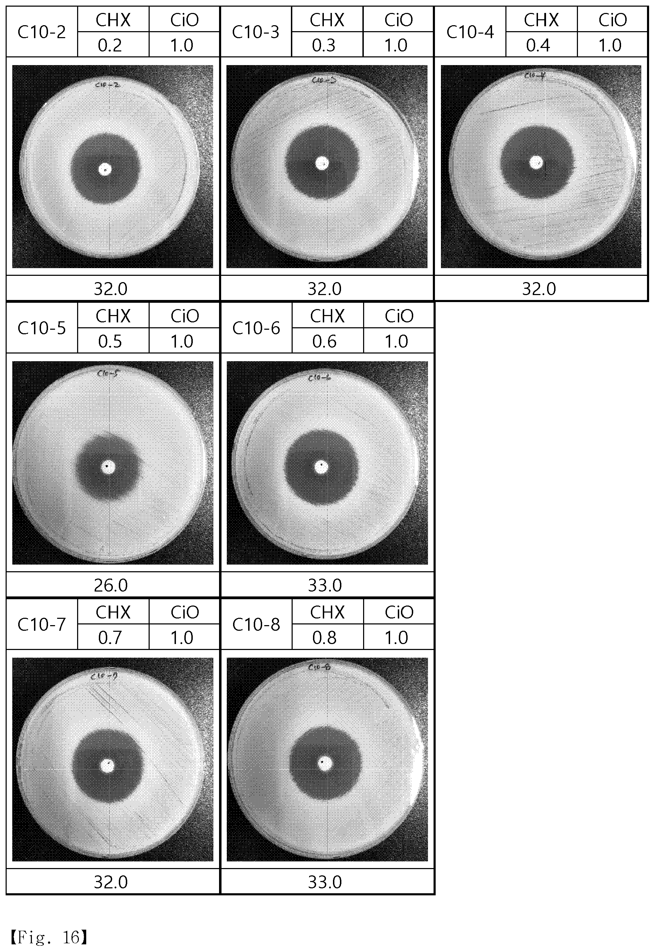

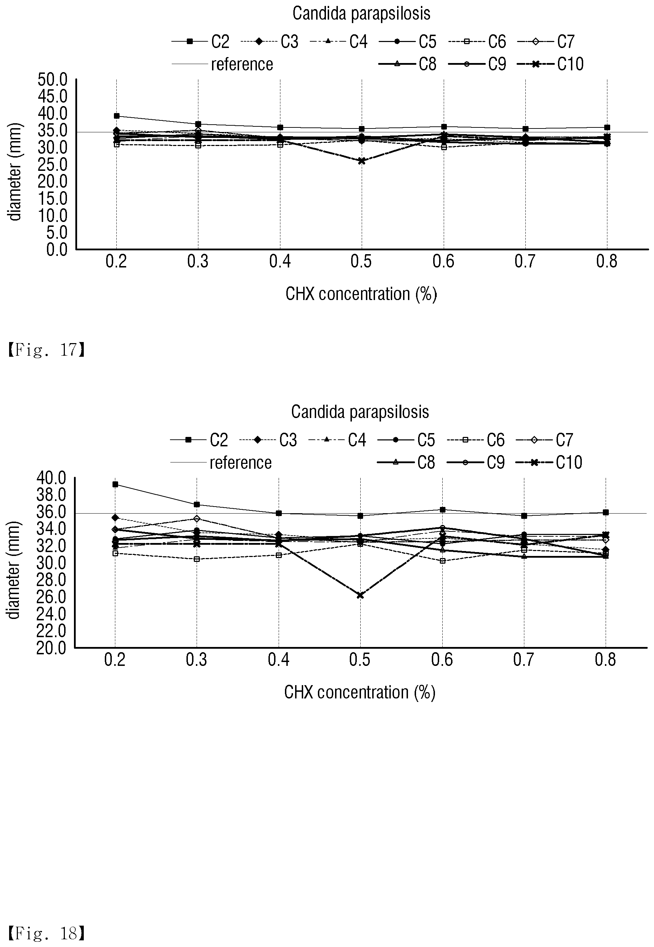

FIGS. 16 and 17 are graphs showing the antimicrobial activity test results of the composition according to the present specification for Candida parapsilosis.

Referring to FIGS. 16 and 17, the size of the antimicrobial activity inhibitory band of the existing product is indicated by a red line to facilitate the comparative analysis. FIG. 17 is an enlarged graph of a portion of an inhibitory band (y-axis) in FIG. 16.

In the Candida parapsilosis, the inhibitory band of the existing product was found to be 34mm, and unlike C. albicans, only the sample of 0.2% CiO concentrations showed a larger inhibitory band than the existing product, and the size of the inhibitory band did not show an increasing tendency depending on the CiO concentrations. However, in the C2 and C3 samples, the size of the inhibitory band tended to decrease as the CHX concentrations increased, but the rest of the sample showed a small error in the tendency to decrease the inhibitory band according to the increase of the CHX concentrations.

### 3. Candida krusei

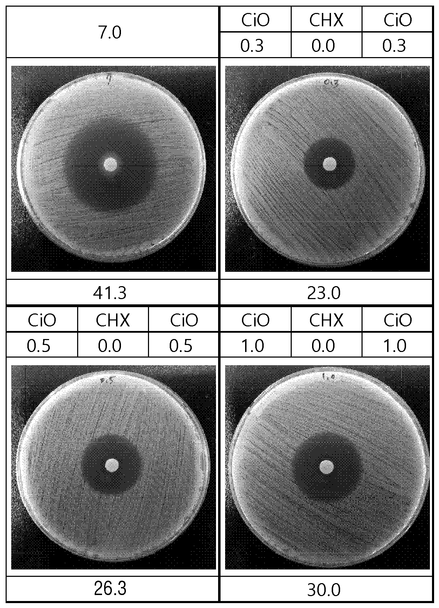

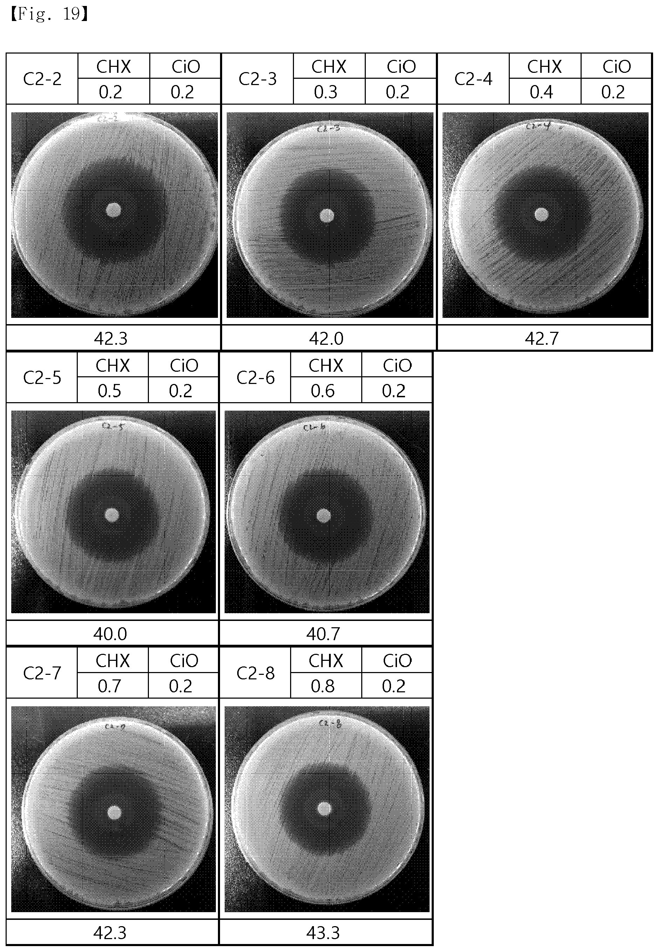

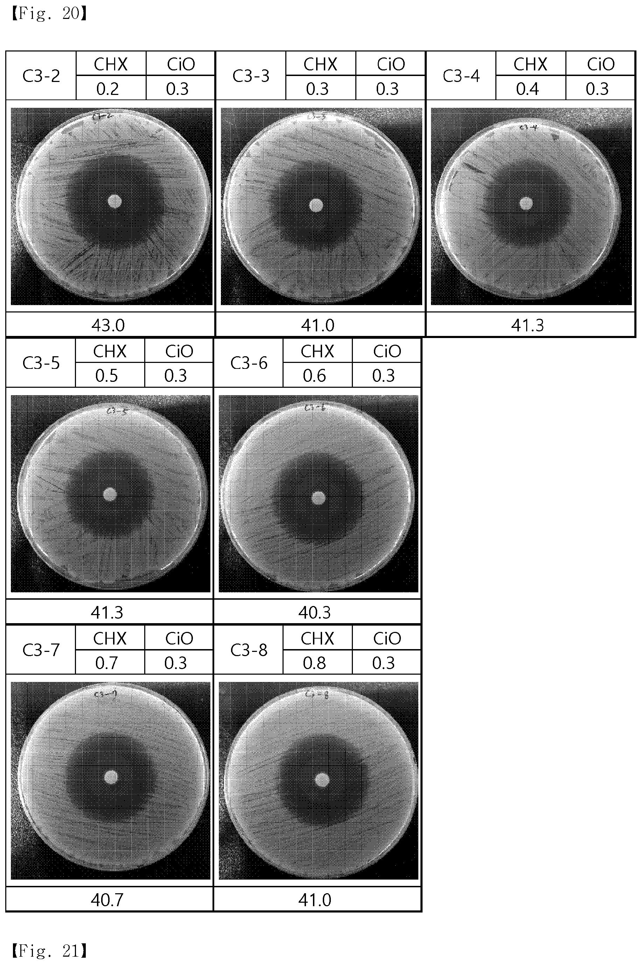

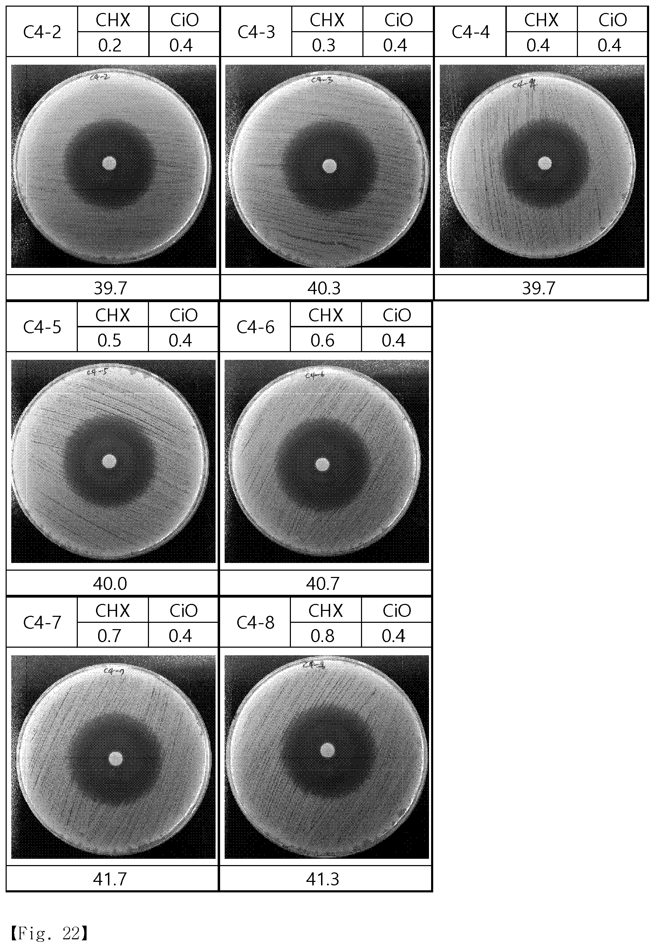

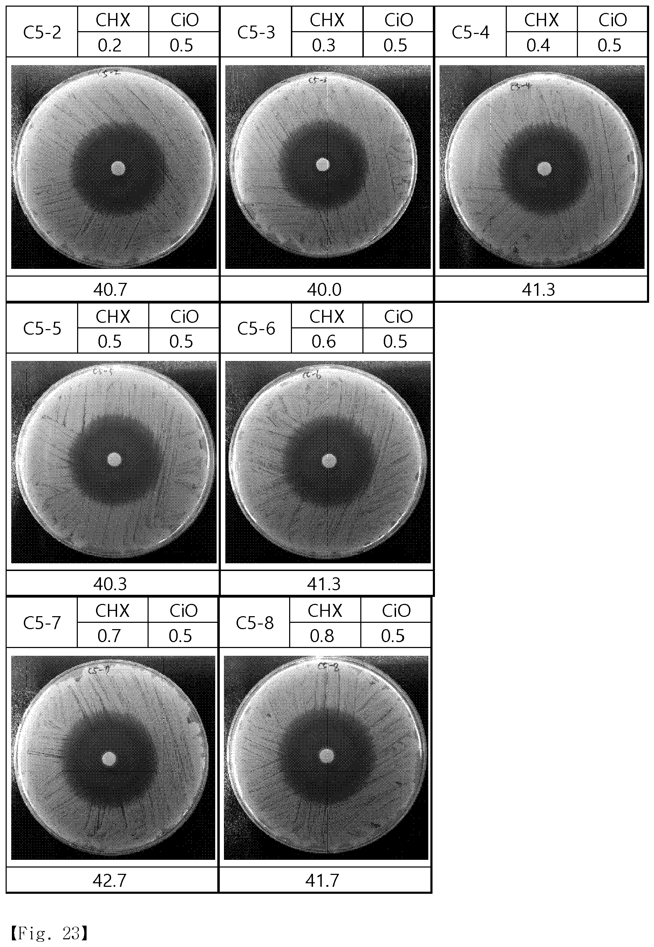

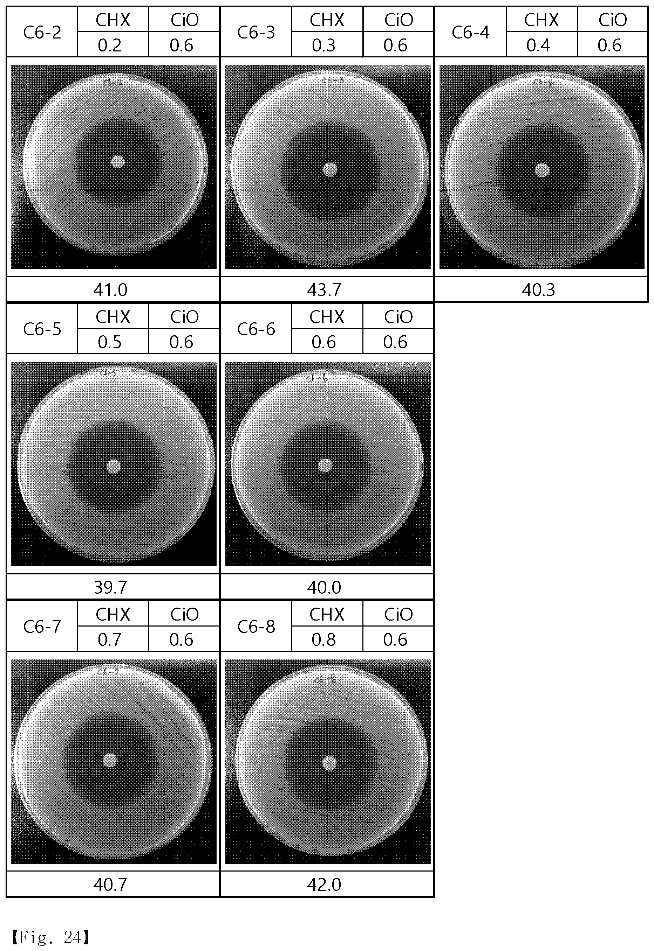

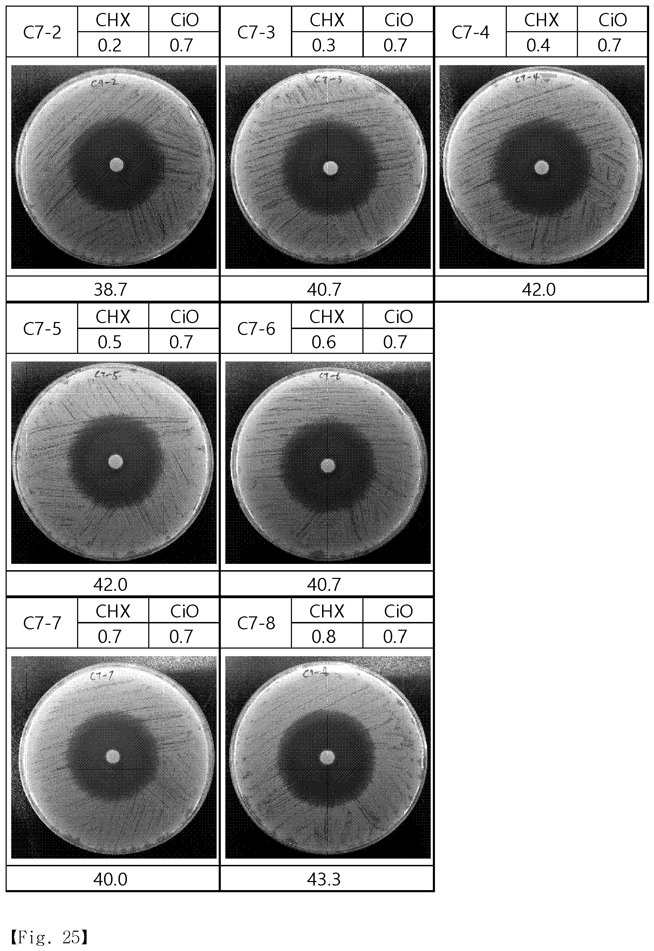

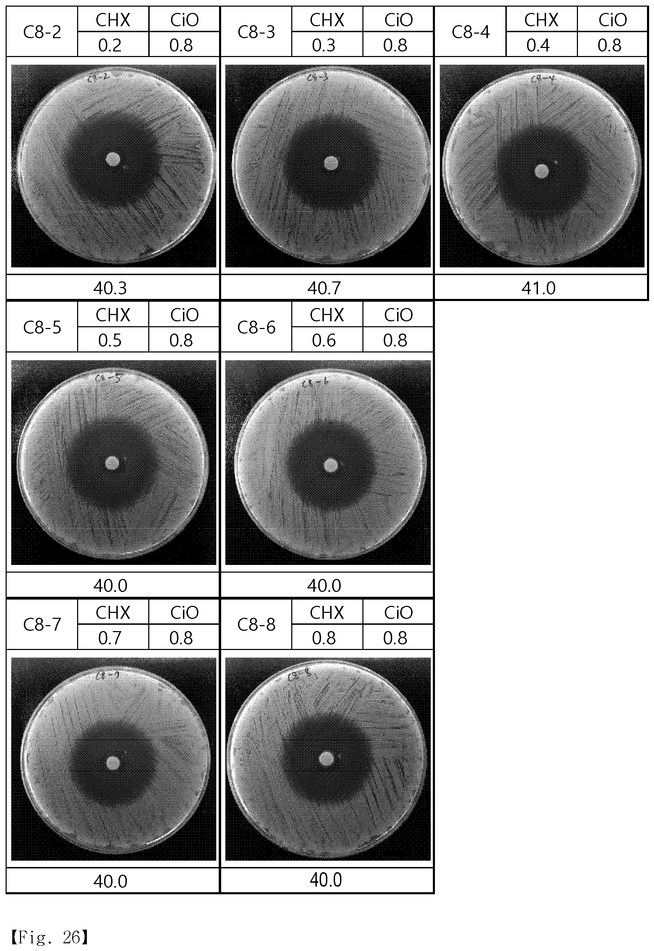

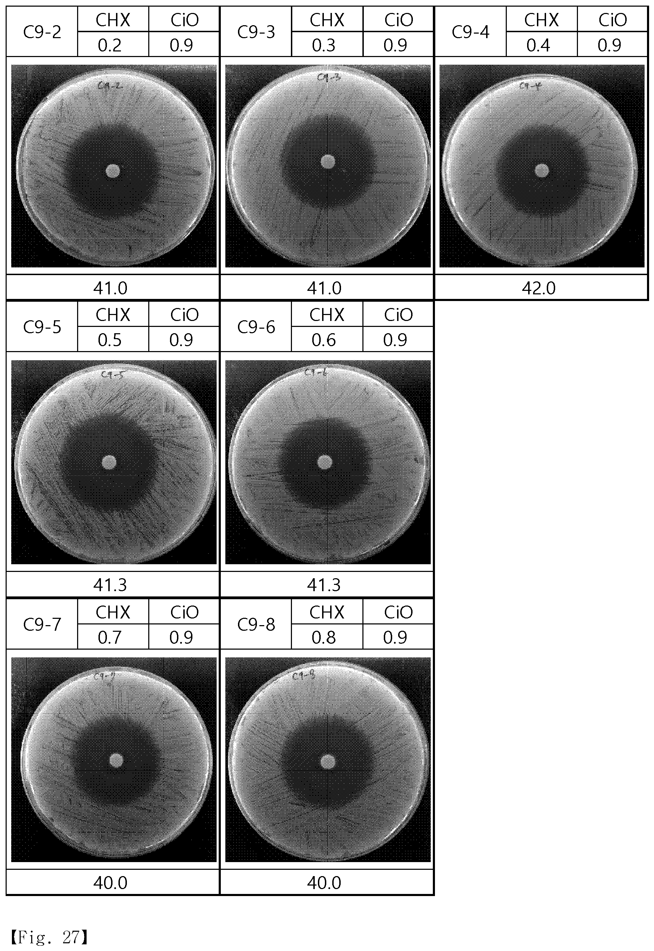

FIGS. 18 to 27 are results of the formation of the inhibitory band, which are antimicrobial activity test results of the composition according to the present specification for Candida krusei.

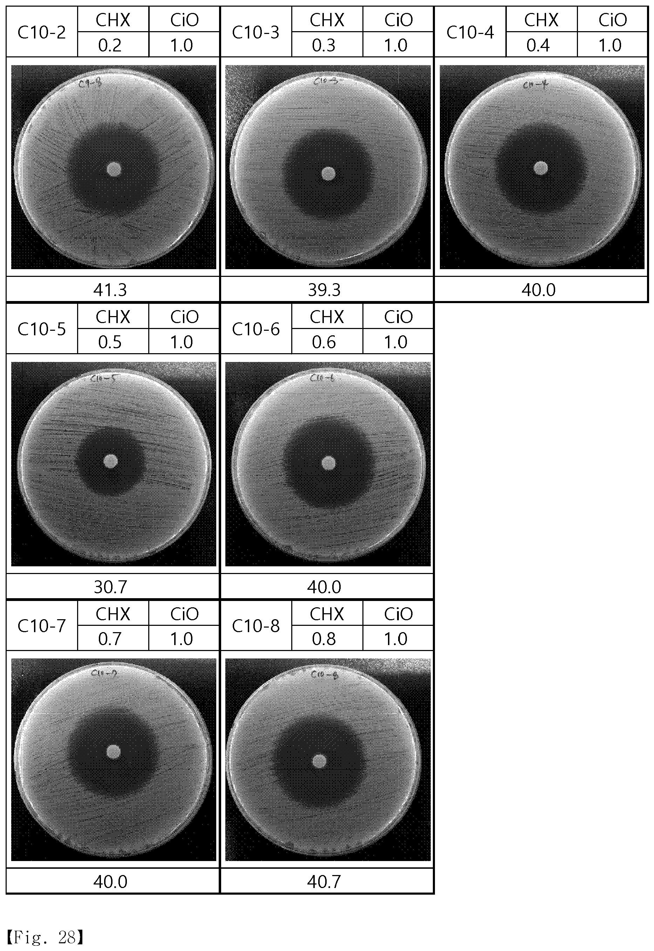

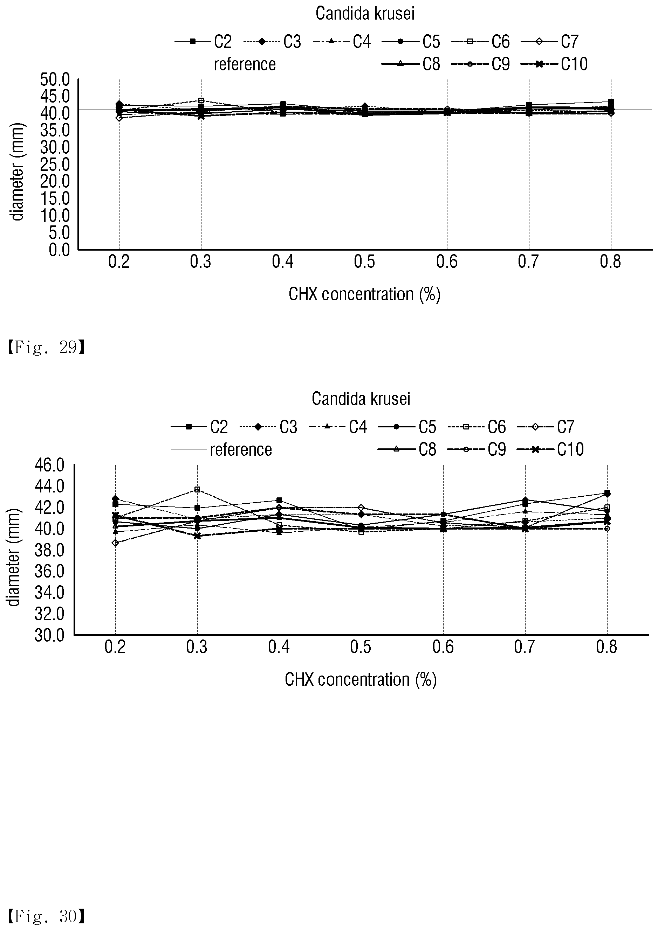

FIGS. 28 and 29 are graphs showing the antimicrobial activity test results of the composition according to the present specification for Candida krusei.

Referring to FIGS. 28 and 29, the size of the antimicrobial activity inhibitory band of the existing product is indicated by a red line to facilitate the comparative analysis. FIG. 29 is an enlarged graph of a portion of an inhibitory band (y-axis) in FIG. 28.

In the Candida krusei, the inhibitory band of the existing product was found to be 41.3 mm, and it was confirmed that no new sample showed a large error with the existing product as a whole. (Because the size of the inhibitory band is measured by the experimenter's hand, it is judged to be an error range from 2 to 3mm.) Of particular note, C2 and C3 samples with low CiO concentrations showed relatively high antimicrobial activity inhibitory bands, and in the range of 0.4 - 0.6% CHX concentrations, the size of the inhibitory band decreases and then increases again from 0.7%.

### 4. Aspergillus fumigatus

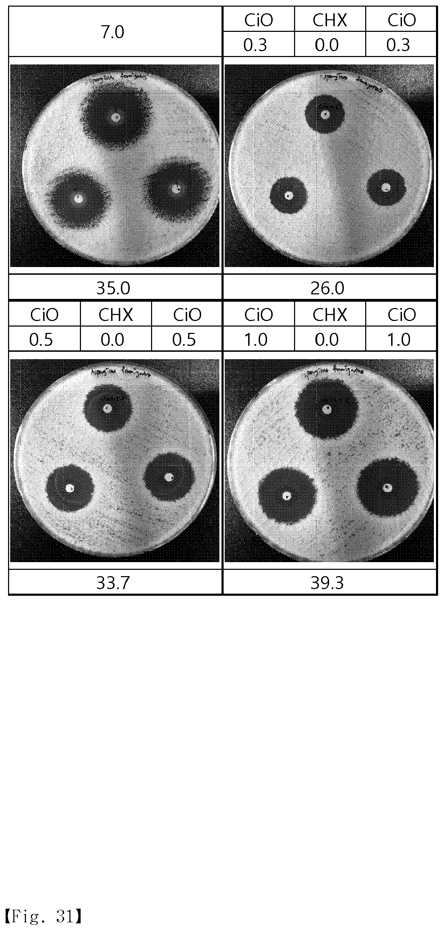

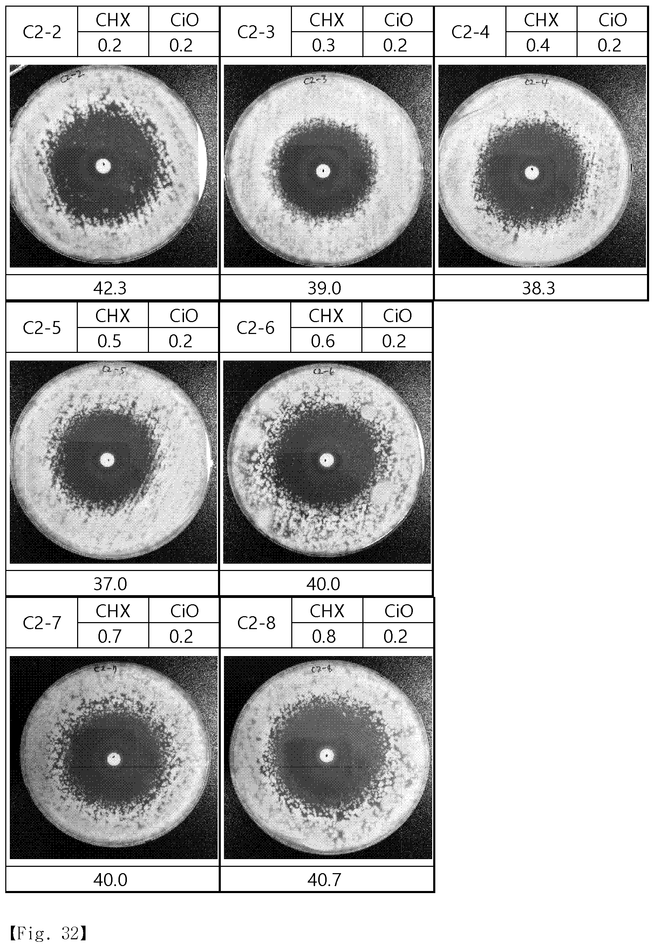

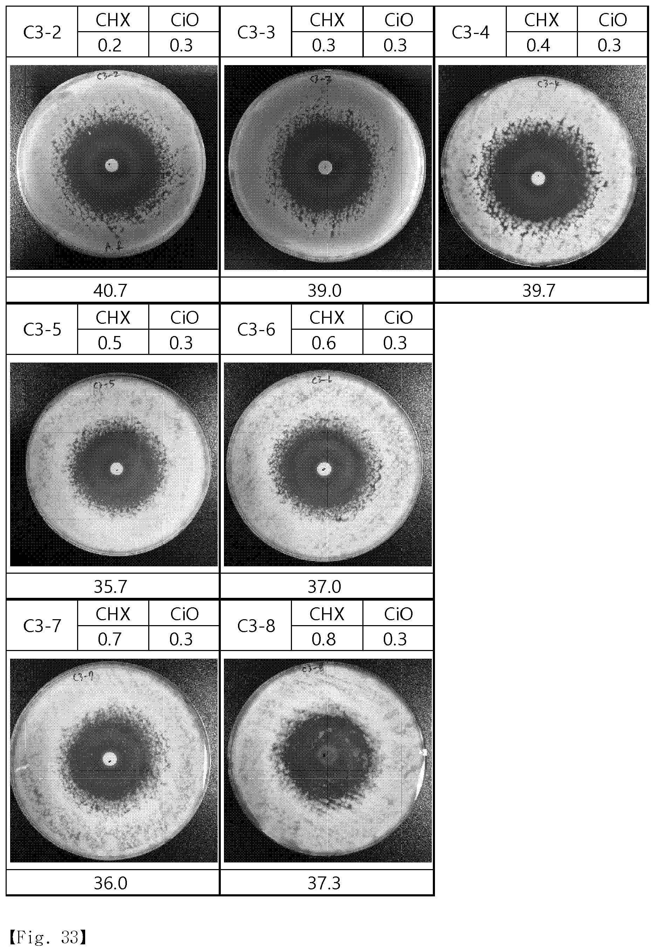

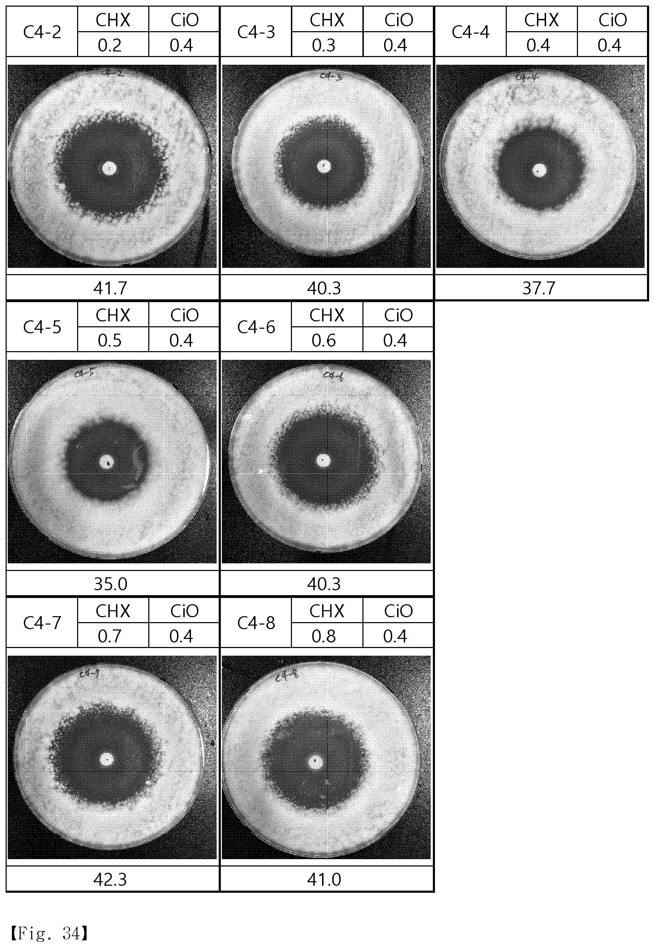

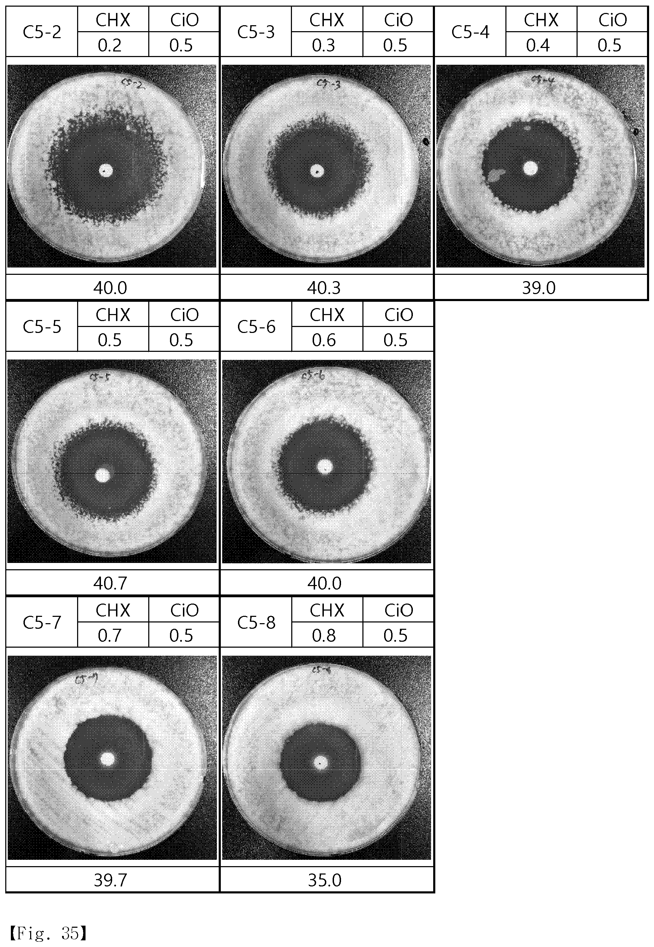

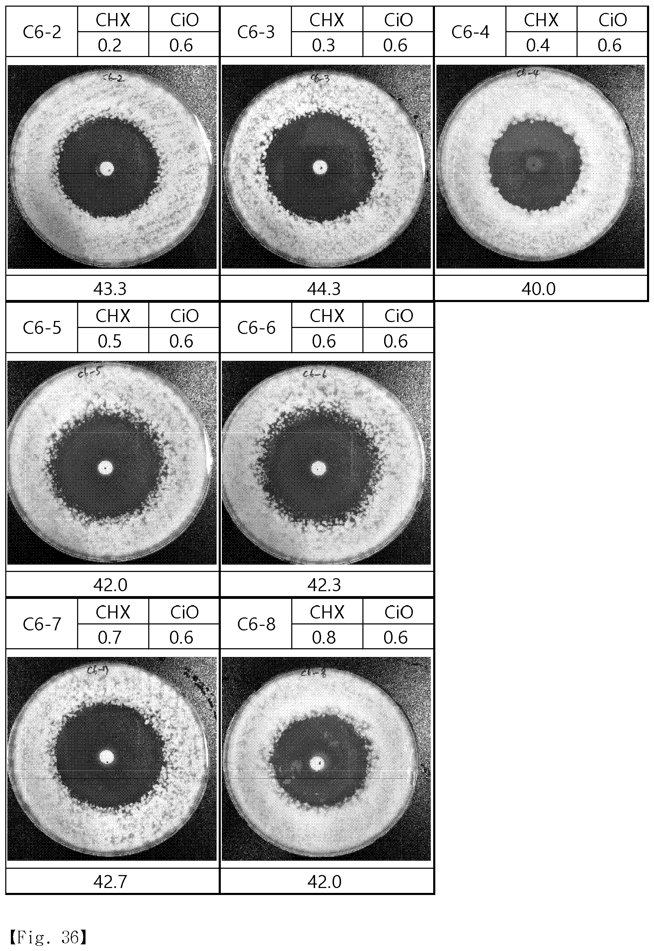

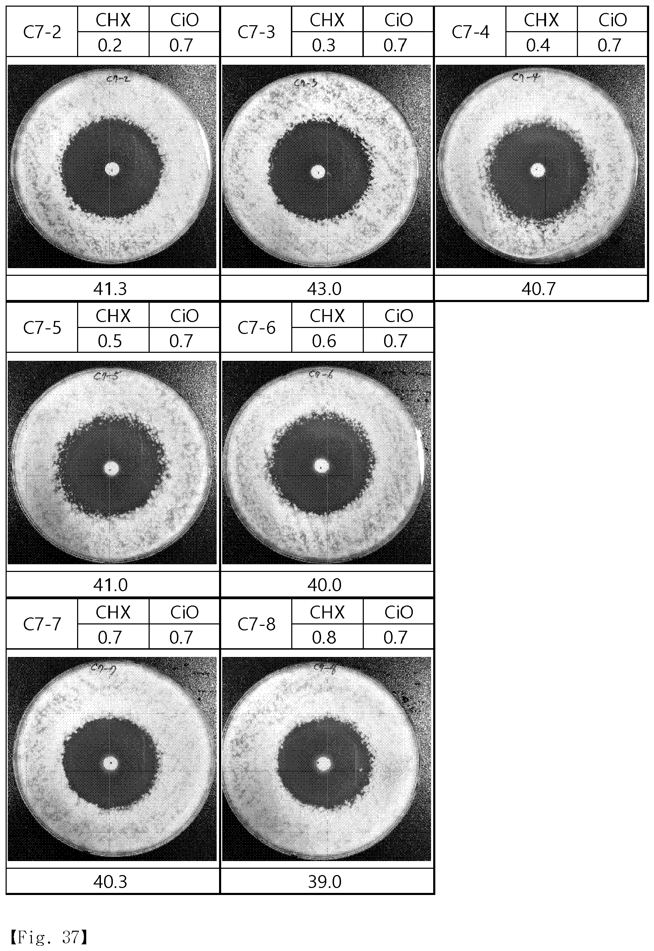

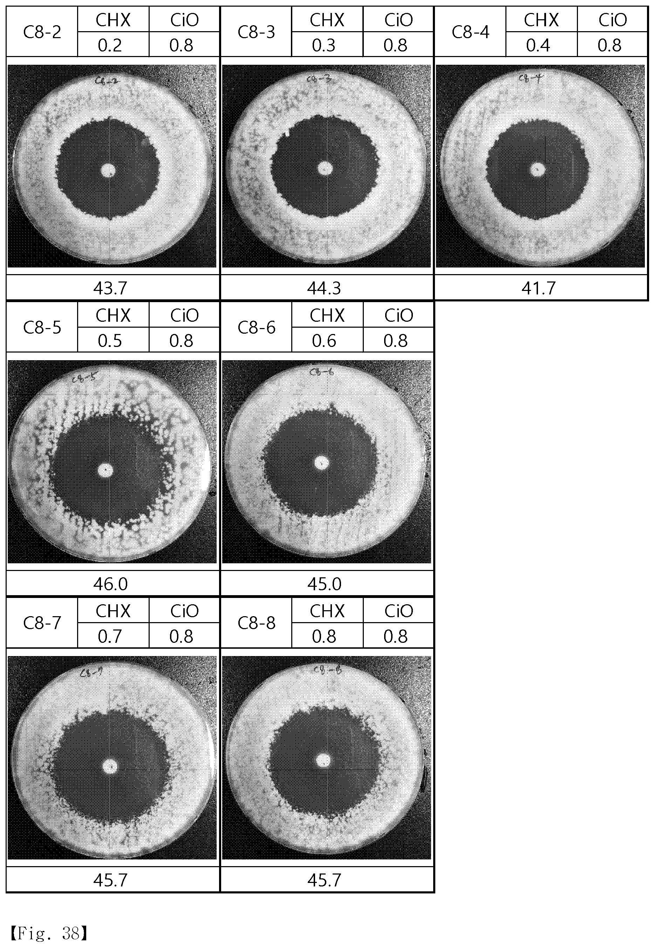

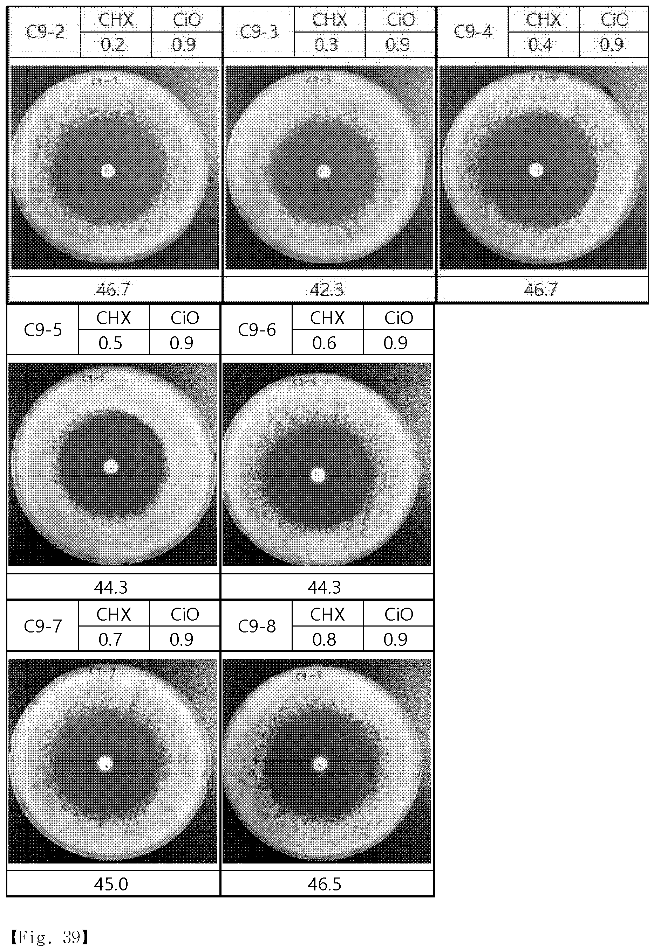

FIGS. 30 to 39 are results of the formation of the inhibitory band, which are the antimicrobial activity test results for Aspergillus fumigatus of the composition according to the present specification.

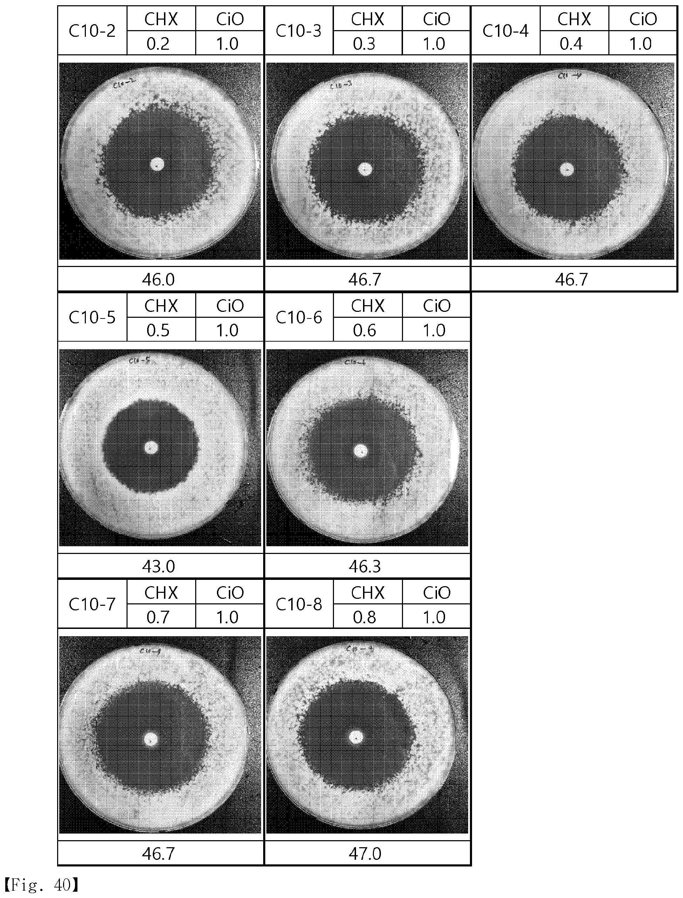

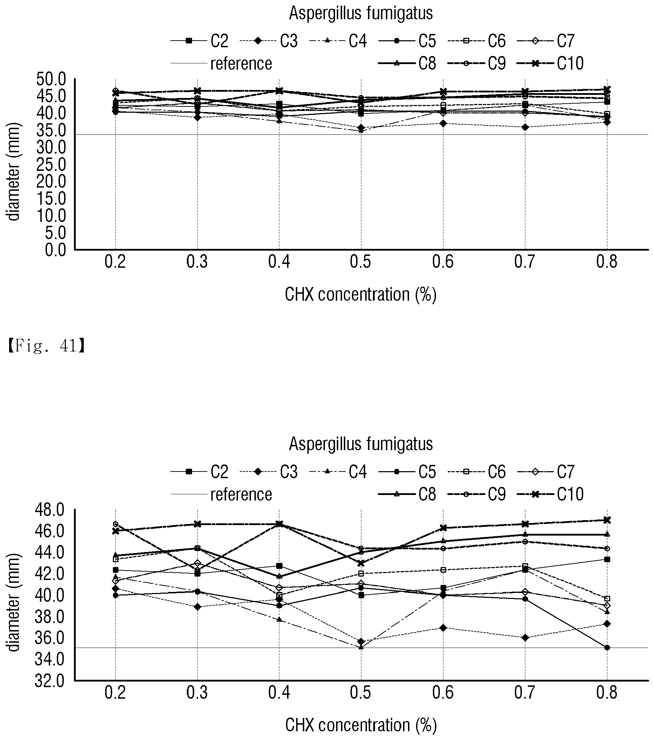

FIGS. 40 and 41 are graphs showing the antimicrobial activity test results of the composition according to the present specification for Aspergillus fumigatus.

Referring to FIGS. 40 and 41, the size of the active bacteria inhibitory band of the existing product is indicated by a red line to facilitate the comparative analysis. FIG. 41 is an enlarged graph of a portion of an inhibitory band (y-axis) in FIG. 40.

Aspergillus fumigatus is a disease-causing agent that infects animals' ears, nose and respiratory system. In the Aspergillus fumigatus, the inhibitory band of the existing product was found to be 35 mm, and the new sample showed overall higher antimicrobial activity than the existing product. Antimicrobial activity was high in C10 sample with the highest CiO concentrations, and the overall sample showed a tendency to increase antimicrobial activity depending on CiO concentrations. The effect of CHX concentrations was mainly observed in samples with CiO concentrations of 0.6% or less, but there was no significant error in the reduction due to 1-2mm difference when comparing 0.2% and 0.8%.

### 5. Precipitate Generation Prevention Experiment

As described above, most liquid medicines include excipients including chloride ions, and the composition of the present invention in combination with terbinafine hydrochloride may also cause precipitation. However, it may further include malic acid and sodium borate to prevent the generation of precipitates of terbinafine hydrochloride. Hereinafter, a description will be given of the precipitate generation experiments of terbinafine hydrochloride.

First, a liquid formulation containing terbinafine hydrochloride, chlorhexidine gluconate and ciclopirox olamine is prepared as shown in the following [Table 3]. Malic acid or polycarboxylic acid other than malic acid is added to the prepared liquid formulation, and the presence or absence of a precipitate is observed.

**[Table 3]**

| Ingredient | Content(%) |
|---|---|
| ethanol | 54.0 |
| purified water | 35.0 |
| chlorhexidine gluconate | 2.0 |
| ciclopirox olamine | 0.5 |
| terbinafine hydrochloride | 1.0 |
| Total | 100.0 |

First, malic acid, citric acid, oxalic acid and tartaric acid are prepared as a polycarboxylic acid such as malic acid. To the liquid formulation prepared in the above [Table 3], polycarboxylic acid was added in 0.1, 0.3, 0.5, 0.7, 1.0, 2.0, 3.0, 4.0, 5.0 (w/v%), respectively, and then chlorhexidine gluconate is adjusted to a stable pH of 5.0 using a pH adjusting agent. And, the liquid formulations were stored under accelerated conditions of 40 ± 2 ° C and relative humidity of 75 ± 5% to observe the presence or absence of precipitates.

The precipitate generation results according to the types of the polycarboxylic acid and the pH adjusting agent are shown in the following [Table 4].

**[Table 4]**

| Polycarboxylic acid | pH adjusting agent | Polycarboxylic Acid Concentrations (%) | | | | | | | | |
|---|---|---|---|---|---|---|---|---|---|---|
| | | 0.1 | 0.3 | 0.5 | 0.7 | 1.0 | 2.0 | 3.0 | 4.0 | 5.0 |
| Malic acid | Sodium borate | O | O | X | X | X | X | O | O | O |
| | Sodium chloride | O | O | O | O | O | O | O | O | O |
| | Ammonia | O | O | O | O | O | O | O | O | O |
| | Sodium citrate | O | O | O | O | O | O | O | O | O |
| | Ammonium carbonate | O | O | O | O | O | O | O | O | O |
| | pH adjustment x | O | O | O | O | O | O | O | O | O |
| Citric acid | Sodium borate | O | O | O | O | O | O | O | O | O |
| | Sodium chloride | O | O | O | O | O | O | O | O | O |
| | Ammonia | O | O | O | O | O | O | O | O | O |
| | Sodium citrate | O | O | O | O | O | O | O | O | O |
| | Ammonium carbonate | O | O | O | O | O | O | O | O | O |
| | pH adjustment x | O | O | O | O | O | O | O | O | O |
| Oxalic acid | Sodium borate | O | O | O | O | O | O | O | O | O |
| | Sodium chloride | O | O | O | O | O | O | O | O | O |
| | Ammonia | O | O | O | O | O | O | O | O | O |
| | Sodium citrate | O | O | O | O | O | O | O | O | O |
| | Ammonium carbonate | O | O | O | O | O | O | O | O | O |
| | pH adjustment x | O | O | O | O | O | O | O | O | O |
| Tartaric acid | Sodium borate | O | O | O | O | O | O | O | O | O |
| | Sodium chloride | O | O | O | O | O | O | O | O | O |
| | Ammonia | O | O | O | O | O | O | O | O | O |
| | Sodium citrate | O | O | O | O | O | O | O | O | O |
| | Ammonium carbonate | O | O | O | O | O | O | O | O | O |
| | pH adjustment x | O | O | O | O | O | O | O | O | O |

| | | | | | | | | | | |
|---|---|---|---|---|---|---|---|---|---|---|
| * Whether or not a precipitate is generated O: Generated X: Non-Generated | | | | | | | | | | |

Referring to the above [Table 4], it can be seen that the liquid formulation containing maleic acid as the polycarboxylic acid and sodium borate as the pH adjusting agent does not generate precipitates.

Next, the content of sodium borate and the pH of the liquid formulation were adjusted and the experiment was performed on whether or not precipitates were generated in the same manner as in [Table 4]. Malic acid was added at 1.0%, the content of sodium borate and the pH of the liquid formulation were adjusted, and a range, in which no precipitate is generated, was examined. The experimental results are shown in the following [Table 5].

**[Table 5]**

| Polycarboxylic acid | pH adjusting agent | Sodium borate dosage (% by weight) | | | | | | |
|---|---|---|---|---|---|---|---|---|
| | | 0.0 | 0.1 | 0.3 | 0.5 | 0.7 | 0.9 | 1.1 |
| Malic acid | pH | 4.6 | 4.9 | 5.3 | 5.7 | 6.0 | 6.4 | 6.6 |
| | result | O | O | X | X | O | O | O |

| | | | | | | | | |
|---|---|---|---|---|---|---|---|---|
| 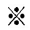 Whether or not a precipitate is generated O: Generated X: Non-Generated | | | | | | | | |

Referring to the above [Table 5], it can be seen that a precipitate is not generated when the pH of the liquid formulation including maleic acid is adjusted to 5.3 or 5.7 by adding 0.3 wt% to 0.5 wt% of sodium borate.

Next, after adding 1.0% of malic acid and 0.3% of sodium borate, an experiment is performed to determine whether precipitates are generated by adjusting the content of chlorhexidine gluconate, terbinafine hydrochloride, and ciclopirox olamine. The experimental results are shown in the following [Table 6] and [Table 7], and experiments were performed on whether or not precipitates of the liquid formulations prepared without adding malic acid and sodium borate are generated were shown in [Table 8].

**[Table 6]**

| Concentrations(%) | 0.1 | 0.3 | 0.5 | 1.0 | 2.0 | 3.0 | 4.0 | 5.0 | 6.0 |
|---|---|---|---|---|---|---|---|---|---|
| Chlorhexidine Gluconate | X | X | X | X | X | X | X | O | O |
| Terbinafine hydrochloride | X | X | X | X | X | O | O | O | O |

| | | | | | | | | | |
|---|---|---|---|---|---|---|---|---|---|
| 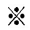 Whether or not a precipitate is generated O: Generated X: Non-Generated | | | | | | | | | |

**[Table 7]**

| | | | | | | | | | |
|---|---|---|---|---|---|---|---|---|---|
| Chlorhexidine Gluconate (wt%) | 0.1 | 0.1 | 0.1 | 0.1 | 0.1 | 0.1 | 0.1 | 0.1 | 0.1 |
| Terbinafine hydrochloride (wt%) | 0.1 | 0.1 | 0.1 | 0.1 | 0.1 | 0.1 | 0.1 | 0.1 | 0.1 |
| Ciclopirox Olamine (wt%) | 0.1 | 0.3 | 0.5 | 1.0 | 2.0 | 3.0 | 4.0 | 5.0 | 6.0 |
| Ethanol (wt%) | 59 | 59 | 59 | 59 | 59 | 59 | 59 | 59 | 59 |
| Purified water (wt%) | suitable amount | suitable amount | suitable amount | suitable amount | suitable amount | suitable amount | suitable amount | suitable amount | suitable amount |
| Total | 100 | | | | | | | | |
| pH | 5.3∼5.7 | | | | | | | | |
| Result | X | X | X | X | X | X | X | X | X |

| | | | | | | | | | |
|---|---|---|---|---|---|---|---|---|---|
| 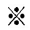 Whether or not a precipitate is generated O: Generated X: Non-Generated | | | | | | | | | |

**[Table 8]**

| | | | | | | | | | |
|---|---|---|---|---|---|---|---|---|---|
| Chlorhexidine Gluconate (wt%) | 0.1 | 0.1 | 0.1 | 0.1 | 0.1 | 0.1 | 0.1 | 0.1 | 0.1 |
| Terbinafine hydrochloride (wt%) | 0.1 | 0.1 | 0.1 | 0.1 | 0.1 | 0.1 | 0.1 | 0.1 | 0.1 |
| Ciclopirox Olamine (wt%) | 0.1 | 0.2 | 0.3 | 0.4 | 0.5 | 0.6 | 0.7 | 0.8 | 0.9 |
| Ethanol (wt%) | 59 | 59 | 59 | 59 | 59 | 59 | 59 | 59 | 59 |
| Purified water (wt%) | suitable amount | suitable amount | suitable amount | suitable amount | suitable amount | suitable amount | suitable amount | suitable amount | suitable amount |
| Total | 100 | | | | | | | | |
| pH | adjustment x | | | | | | | | |
| Result | O | O | O | O | O | O | O | O | O |

| | | | | | | | | | |
|---|---|---|---|---|---|---|---|---|---|
| 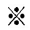 Whether or not a precipitate is generated O: Generated X: Non-Generated | | | | | | | | | |

Referring to Tables 6 to 8 above, in the liquid formulation containing maleic acid and sodium borate and having the pH of 5.3 to 5.7, it can be seen that when chlorhexidine gluconate is 0.1 wt%, terbinafne hydrochloride is 0.1 wt% and ciclopirox olamine is 0.1 wt% to 6.0 wt%, no precipitate is generated.

On the other hand, in liquid formulations that do not contain malic acid and sodium borate and do not have pH control, it can be seen that when chlorhexidine gluconate is 0.1 wt%, terbinafine hydrochloride is 0.1 wt%, and ciclopirox olamine is 0.1 wt% to 0.9 wt%, no precipitate is generated.

Therefore, in a liquid formulation having 2.0 wt% of chlorohexidine gluconate, 1.0 wt% of terbinafine hydrochloride, and 0.5 wt% of ciclopirox olamine, sodium borate as a pH adjusting agent along with malic acid may be added to prevent the generation of precipitates. The content of the malic acid and sodium borate is 0.5 to 2.0 wt% and 0.3 to 0.5 wt%, respectively, and the pH value range from 5.3 to 5.7 is proper. Further, in the liquid formulation prepared by the above method, it can be seen that the range of chlorhexidine gluconate, in which no precipitate is generated, is 0.1 to 4.0 wt%, and the concentrations of terbinafine hydrochloride is 0.1 to 2.0 wt%.

## Claims

1. A pharmaceutical composition for use in preventing or treating skin infections comprising:
terbinafine hydrochloride 0.1 to 2.0 parts by weight;
chlorhexidine gluconate 0.1 to 4.0 parts by weight,
ciclopirox olamine 0.1 to 6.0 parts by weight,
malic acid 0.5 to 2.0 parts by weight; and
sodium borate 0.3 to 0.5 parts by weight, based on the pharmaceutical composition,
wherein the composition has a pH value in the range of 5.3 to 5.7.

2. The pharmaceutical composition for use in preventing or treating skin infections of claim 1, further comprising,
tea tree oil.

3. The pharmaceutical composition for use in preventing or treating skin infections of claim 2,
wherein the tea tree oil is included in an amount of 0.1 to 10.0 parts by weight based on the composition.

4. The pharmaceutical composition for use in preventing or treating skin infections of claim 2, further comprising
an oat extract.

5. The pharmaceutical composition for use in preventing or treating skin infections of claim 4,
wherein the oat extract is included in an amount of 0.1 to 10.0 parts by weight based on the composition.

6. The pharmaceutical composition for use in preventing or treating skin infections of claim 4, further comprising
any one selected from a group including phytosphingosine and a mixture of phytosphingosine and ceramide.

7. The pharmaceutical composition for use in preventing or treating skin infections of claim 1,
wherein the composition is used for an animal.

8. The pharmaceutical composition for use in preventing or treating skin infections of claim 1, further comprising: any one selected from a group including tea tree oil, a mixture of tea tree oil and oat extract, a mixture of tea tree oil, oat extract and phytosphingosine, and a mixture of tea tree oil, oat extract, phytosphingosine and ceramide.

9. A cosmetic composition for preventing or improving skin infections comprising:
terbinafine hydrochloride 0.1 to 2.0 parts by weight,
chlorhexidine gluconate 0.1 to 4.0 parts by weight,
ciclopirox olamine 0.1 to 6.0 parts by weight,
malic acid 0.5 to 2.0 parts by weight; and
sodium borate 0.3 to 0.5 parts by weight, based on the pharmaceutical composition,
wherein the composition has a pH value in the range of 5.3 to 5.7.

10. The cosmetic composition for preventing or improving skin infections of claim 9, further comprising,
any one selected from a group including tea tree oil, a mixture of tea tree oil and oat extract, a mixture of tea tree oil, oat extract and phytosphingosine, and a mixture of tea tree oil, oat extract, phytosphingosine and ceramide.

11. The cosmetic composition for preventing or improving skin infections of claim 9, wherein formulation of the composition is a lotion, cream, ointment, shampoo, spray, gel or shampoo.

## Patentansprüche

1. Eine pharmazeutische Zusammensetzung zur Verwendung bei der Vorbeugung oder Behandlung von Hautinfektionen, umfassend:
Terbinafinhydrochlorid zu 0,1 bis 2,0 Gewichtsteilen;
Chlorhexidingluconat zu 0,1 bis 4,0 Gewichtsteilen;
Ciclopiroxolamin zu 0,1 bis 6,0 Gewichtsteilen;
Äpfelsäure zu 0,5 bis 2,0 Gewichtsteilen; und
Natriumborat zu 0,3 bis 0,5 Gewichtsteilen, basierend auf der pharmazeutischen Zusammensetzung,
wobei die Zusammensetzung einen pH-Wert in dem Bereich von 5,3 bis 5,7 aufweist.

2. Die pharmazeutische Zusammensetzung zur Verwendung bei der Vorbeugung oder Behandlung von Hautinfektionen nach Anspruch 1, ferner umfassend,
Teebaumöl.

3. Die pharmazeutische Zusammensetzung zur Verwendung bei der Vorbeugung oder Behandlung von Hautinfektionen nach Anspruch 2,
wobei das Teebaumöl in einer Menge zu 0,1 bis 10,0 Gewichtsteilen eingeschlossen ist, basierend auf der Zusammensetzung.

4. Die pharmazeutische Zusammensetzung zur Verwendung bei der Vorbeugung oder Behandlung von Hautinfektionen nach Anspruch 2, ferner umfassend
einen Haferextrakt.

5. Die pharmazeutische Zusammensetzung zur Verwendung bei der Vorbeugung oder Behandlung von Hautinfektionen nach Anspruch 4,
wobei der Haferextrakt in einer Menge zu 0,1 bis 10,0 Gewichtsteilen eingeschlossen ist, basierend auf der Zusammensetzung.

6. Die pharmazeutische Zusammensetzung zur Verwendung bei der Vorbeugung oder Behandlung von Hautinfektionen nach Anspruch 4, ferner umfassend
ein beliebiges ausgewählt aus einer Gruppe, die Phytosphingosin und eine Mischung aus Phytosphingosin und Ceramid einschließt.

7. Die pharmazeutische Zusammensetzung zur Verwendung bei der Vorbeugung oder Behandlung von Hautinfektionen nach Anspruch 1,
wobei die Zusammensetzung für ein Tier verwendet wird.

8. Die pharmazeutische Zusammensetzung zur Verwendung bei der Vorbeugung oder Behandlung von Hautinfektionen nach Anspruch 1, ferner umfassend: ein beliebiges ausgewählt aus einer Gruppe, die Teebaumöl, eine Mischung aus Teebaumöl und Haferextrakt, eine Mischung aus Teebaumöl, Haferextrakt und Phytosphingosin, und eine Mischung aus Teebaumöl, Haferextrakt, Phytosphingosin und Ceramid einschließt.

9. Eine kosmetische Zusammensetzung zum Vorbeugen oder Verbessern von Hautinfektionen, umfassend:
Terbinafinhydrochlorid zu 0,1 bis 2,0 Gewichtsteilen;
Chlorhexidingluconat zu 0,1 bis 4,0 Gewichtsteilen;
Ciclopiroxolamin zu 0,1 bis 6,0 Gewichtsteilen;
Äpfelsäure zu 0,5 bis 2,0 Gewichtsteilen; und
Natriumborat zu 0,3 bis 0,5 Gewichtsteilen, basierend auf der pharmazeutischen Zusammensetzung,
wobei die Zusammensetzung einen pH-Wert in dem Bereich von 5,3 bis 5,7 aufweist.

10. Die kosmetische Zusammensetzung zum Vorbeugen oder Verbessern von Hautinfektionen nach Anspruch 9, ferner umfassend,
ein beliebiges ausgewählt aus einer Gruppe, die Teebaumöl, eine Mischung aus Teebaumöl und Haferextrakt, eine Mischung aus Teebaumöl, Haferextrakt und Phytosphingosin, und eine Mischung aus Teebaumöl, Haferextrakt, Phytosphingosin und Ceramid einschließt.

11. Die kosmetische Zusammensetzung zum Vorbeugen oder Verbessern von Hautinfektionen nach Anspruch 9, wobei die Formulierung der Zusammensetzung ein/eine Lotion, Creme, Salbe, Shampoo, Spray, Gel oder Shampoo ist.

## Revendications

1. Composition pharmaceutique destinée à être utilisée dans la prévention ou le traitement d'infections cutanées comprenant :
du chlorhydrate de terbinafine 0,1 à 2,0 parties en poids ;
du gluconate de chlorhexidine 0,1 à 4,0 parties en poids ;
de la ciclopirox olamine 0,1 à 6,0 parties en poids ;
de l'acide malique 0,5 à 2,0 parties en poids ; et
du borate de sodium 0,3 à 0,5 partie en poids, sur la base de la composition pharmaceutique,
dans laquelle la composition a une valeur de pH comprise dans la plage de 5,3 à 5,7.

2. Composition pharmaceutique destinée à être utilisée dans la prévention ou le traitement d'infections cutanées selon la revendication 1, comprenant en outre,
de l'huile d'arbre à thé.

3. Composition pharmaceutique destinée à être utilisée dans la prévention ou le traitement d'infections cutanées selon la revendication 2,
dans laquelle l'huile d'arbre à thé est incluse en une quantité de 0,1 à 10,0 parties en poids sur la base de la composition.

4. Composition pharmaceutique destinée à être utilisée dans la prévention ou le traitement d'infections cutanées selon la revendication 2, comprenant en outre
un extrait d'avoine.

5. Composition pharmaceutique destinée à être utilisée dans la prévention ou le traitement d'infections cutanées selon la revendication 4,
dans laquelle l'extrait d'avoine est inclus en une quantité de 0,1 à 10,0 parties en poids sur la base de la composition.

6. Composition pharmaceutique destinée à être utilisée dans la prévention ou le traitement d'infections cutanées selon la revendication 4, comprenant en outre
l'un quelconque choisi dans un groupe comportant la phytosphingosine et un mélange de phytosphingosine et de céramide.

7. Composition pharmaceutique destinée à être utilisée dans la prévention ou le traitement d'infections cutanées selon la revendication 1,
dans laquelle la composition est utilisée pour un animal.

8. Composition pharmaceutique destinée à être utilisée dans la prévention ou le traitement d'infections cutanées selon la revendication 1, comprenant en outre : l'un quelconque choisi dans un groupe comportant l'huile d'arbre à thé, un mélange d'huile d'arbre à thé et d'extrait d'avoine, un mélange d'huile d'arbre à thé, d'extrait d'avoine et de phytosphingosine, et un mélange d'huile d'arbre à thé, d'extrait d'avoine, de phytosphingosine et de céramide.

9. Composition cosmétique permettant de prévenir ou d'améliorer des infections cutanées comprenant :
du chlorhydrate de terbinafine 0,1 à 2,0 parties en poids ;
du gluconate de chlorhexidine 0,1 à 4,0 parties en poids ;
de la ciclopirox olamine 0,1 à 6,0 parties en poids ;
de l'acide malique 0,5 à 2,0 parties en poids ; et
du borate de sodium 0,3 à 0,5 partie en poids, sur la base de la composition pharmaceutique,
dans laquelle la composition a une valeur de pH comprise dans la plage de 5,3 à 5,7.

10. Composition cosmétique permettant de prévenir ou d'améliorer des infections cutanées selon la revendication 9, comprenant en outre,
l'un quelconque choisi dans un groupe comportant l'huile d'arbre à thé, un mélange d'huile d'arbre à thé et d'extrait d'avoine, un mélange d'huile d'arbre à thé, d'extrait d'avoine et de phytosphingosine, et un mélange d'huile d'arbre à thé, d'extrait d'avoine, de phytosphingosine et de céramide.

11. Composition cosmétique permettant de prévenir ou d'améliorer des infections cutanées selon la revendication 9, dans laquelle la formulation de la composition est une lotion, une crème, une pommade, un shampooing, une pulvérisation, un gel ou un shampooing.
